(19) Europäisches Patentamt

European Patent Office

Office européen des brevets

(11) **EP 1 305 628 B1**

(12) **EUROPEAN PATENT SPECIFICATION**

(45) Date of publication and mention
of the grant of the patent:
**04.05.2005 Bulletin 2005/18**

(51) Int Cl.[7]: **G01N 33/553**, B03C 5/00,
B22F 1/02

(21) Application number: **01946449.4**

(22) Date of filing: **14.06.2001**

(86) International application number:
**PCT/US2001/019357**

(87) International publication number:
**WO 2001/096023 (20.12.2001 Gazette 2001/51)**

(54) **DIELECTRICALLY-ENGINEERED MICROPARTICLES**

DIELEKTRISCH-HERGESTELLTE MIKROPARTIKEL

MICROPARTICULES CONCUES DE MANIERE DIELECTRIQUE

(84) Designated Contracting States:
**AT BE CH CY DE DK ES FI FR GB GR IE IT LI LU
MC NL PT SE TR**

(30) Priority: **14.06.2000 US 211515 P**

(43) Date of publication of application:
**02.05.2003 Bulletin 2003/18**

(73) Proprietor: **BOARD OF REGENTS THE
UNIVERSITY OF TEXAS SYSTEM
Austin, TX 78701 (US)**

(72) Inventors:
• **GASCOYNE, Peter, R., C.**
Bellaire, TX 77401 (US)
• **BECKER, Frederick, F.**
Houston, TX 77024 (US)
• **VYKOUKAL, Jody**
Houston, TX 77030 (US)
• **WANG, Xiao-Bo**
San Diego, CA 92128 (US)

(74) Representative: **Dehmel, Albrecht, Dr.
Dehmel & Bettenhausen
Patentanwälte,
Herzogspitalstrasse 11
80331 München (DE)**

(56) References cited:
**WO-A-95/17258**          **US-A- 5 384 073**

**Description**

**BACKGROUND OF THE INVENTION**

**1. Field of the Invention**

The present invention relates generally to the fields of chemistry and the life-sciences. More particularly, it concerns techniques for the manipulation, separation, purification, and indexing of target analytes using dielectrically-engineered microparticles (referred to herein as DEMPs).

**2. Description of Related Art**

Improved methods for separating and identifying chemicals, cells and biomolecules have been fundamental to many advances in chemistry and the life sciences. Much of the discovery process is based on determining qualitative and quantitative information about a particular chemical, cell, biomolecule or other analyte. Analysis methods such as filtration, centrifugation, spectroscopy and light microscopy typically exploit differences in the inherent physical properties of analytes to achieve analyte separation and/or detection.

Improvements in these basic methods have generally fallen into two categories: (i) the resolving ability, or sensitivity, of the method has been improved to better differentiate subtle physical differences between analytes, or (ii) a substance, or *label,* with certain properties that are readily discernable has been coupled to an analyte to make the analyte detectable or easier to resolve indirectly. Gradient centrifugation and high performance liquid chromatography are examples of methods based on increasing the sensitivity of an existing method; cell-staining with fluorescent antibodies and biomolecule radiolabeling are examples of improved methods based on coupling labels to analytes to facilitate resolution of an analyte. Although these improvements have exhibited degrees of success in the field, problems remain. Notably, these methods still do not allow for a method whereby analytes may be indexed, detected, and manipulated at once, nor do they allow for the separate manipulation of many different types of analytes at once.

One type of traditional analysis that makes use of labels is termed a "one-pot" reaction. One-pot reactions are those where reagents are simply added to a sample aliquot in a single test tube or beaker. Any molecules of target analyte present in the sample react with the added indicator to yield a colorimetric, fluorescent or chemiluminescent product or complex. This reaction product or complex is then detected and, usually, quantified. The defining feature of such methods is that the detectable species exists *only* when the target analyte is actually present in the sample.

One example of a useful label for a one pot analysis is a molecular beacon. Molecular beacons utilize a molecule that has a built in fluorophore and a quencher. The fluorophore and quencher are held in close proximity until such time that molecule is bound to a target. At that time, they are pulled sufficiently farther apart so that the fluorophore can fluoresce. When the quencher no longer quenches, the target can be observed via the fluorescence.

Other examples of one-pot assays include colorimetric pH detection or non-specific labeling of nucleic acids using an intercalating dye, such as ethidium bromide. Techniques such as southern and northern blotting for nucleic acids and ELISA and western blotting for proteins use labeled probes that bind to and facilitate the detection of specific biochemical analytes. These antibody or nucleic acid probes are radioactive, fluorescent, or enzymatically active whether or not they are bound to their target analyte:

In order for the above methods to yield useful results, however, it is necessary to distinguish between the unbound analyte, the free probe, and the analyte-probe complexes. This is accomplished by immobilizing the analyte-probe complexes to a solid support and then washing away the free, unbound molecules, leaving only the labeled analyte-probe complexes attached to the solid support. Unfortunately, this process may be somewhat complicated and time-consuming.

In order to solve at least some of the problems inherent in traditional identification and separation of analytes, certain microparticles have been used. In the late 1970's, techniques were developed that enable relatively straightforward production of uniformly sized microparticles in the submicron to 100-micron size range. Later, techniques for making microparticles having magnetic properties were also developed. Microparticles produced using these techniques have been linked to various probes that interact with or bind to specific target analytes or classes of analytes to form microparticle-analyte complexes. In this way, microparticles can be made to act as labels that are specific for target analytes.

Existing microparticle labels may be divided broadly into two categories, namely those for analyte detection and those for analyte manipulation. In both categories, labels sensitized against a specific target analyte are added to a sample and incubated under conditions that facilitate binding of the target analyte, if present in the sample, to the microparticle-based label to form a microparticle-analyte complex.

In existing analyte detection protocols, certain physical properties of the microparticle, such as fluorescence, opacity to light or other radiation, or emission of radiation have been exploited as reporters to infer the presence of the microparticle-analyte complex. For example, a metallic microparticle or nanoparticle that complexes with a target protein in a cell via an antibody probe can be observed and quantified by electron microscopy and used to infer that the target protein analyte is in specific locations in the cell and is an example of a label used in detection protocols.

In detection protocols, it is not the analyte that is detected directly. Instead, the presence of the analyte is inferred by its association with the microparticle reporter, an association mediated by the interaction of the probe and the analyte. Detection protocols can also include two-step labeling methods in which a secondary label is used to reveal the presence of the microparticle-analyte complex. In this case, the analyte attaches to a first probe on the microparticle and then the analyte is subsequently labeled with a second specific label which is then used as the reporter that allows the presence of the analyte to be inferred. Because the analyte ends up being effectively sandwiched between the microparticle and a second, reporter label, such a double labeling protocols are familiarly termed "sandwich assays" in the art.

In manipulation protocols, microparticle-based labels are used as "handles" to assist in the physical manipulation of analytes. In such protocols, certain physical properties of the microparticle such as density, electrical charge, or size are exploited to isolate, separate or otherwise manipulate the microparticle-analyte complex. In such methods the analyte is not manipulated directly. Instead, the microparticle (i.e., "the handle") is manipulated and any analyte bound to the microparticle, is manipulated indirectly based on its association with the microparticle. Manipulation protocols based on microparticle labels unfortunately require additional analysis steps to identify the target analyte.

Although the above microparticle-based systems have exhibited at least a degree of utility in this field, the necessary additional steps of identifying a target (apart from manipulating the target) represent extra time and cost to the scientist or engineer. Further, even with the use of microparticles, it is sometimes the case that the detection of the microparticle itself does not necessarily infer the presence of the target analyte. Still further, traditional microparticles do not allow for the simultaneous, separate manipulation of many different types of analytes. Simply put, traditional microparticles do not allow for the indexing of different analytes followed by simultaneous manipulation, detection, and/or identification. In other words, traditional techniques do not allow for the creation of a library of different probes that may each bind to different targets and allow for simultaneous manipulation, identification, and detection of the different species.

Gold sol coated with alkanethiols, which provide groups on the sol available for the linking of binding moieties such as antibodies are known from US-A-5 384 073.

Certain problems, weaknesses, or shortcomings mentioned above are not meant to be exhaustive; other problems are know to exist within the art. However, the above discussion demonstrates that a need exists for improved methodology relating to manipulation, separation, purification, and indexing of analytes.

## SUMMARY OF THE INVENTION

The dielectrically-engineered microparticles of the invention are defined by the features of claim 1.

The dielectrically-engineered microparticle labels are defined by claim 4 or claim 5.

A method for detecting a complex is defined by the features of claim 6.

A method for manipulating a complex is defined by claim 9.

A method for identifying one or more complexes within a sample is defined by the features of claim 16.

Dielectrically-engineered microparticle labels made and used according to the present disclosure can be designed to overcome limitations discussed above because analyte indexing may be achieved. In particular, the present disclosure allows for the simultaneous identification, manipulation, and detection of a variety of different target analytes through the use of a library of DEMPs having different, distinguishable dielectric properties. Alternatively, the present disclosure overcomes limitations in the art because it allows analyte binding to be detected. Specifically, microparticle labels with dielectric properties that are sensitive to analyte binding can be used to confirm analyte binding by sensing the change in the AC electrokinetic behavior of the label following the binding.

Uses for dielectrically-engineered microparticles according to embodiments disclosed herein are vast and include, but are not limited to, blood analysis; disease detection and characterization; clinical preparation of pure cell populations; the detection and identification of pathogens in food processing, public water distribution systems, agriculture, and the environment; the separation of subcellular compartments, the purification of stem cells for bone marrow transplants, and the purging or collection of diseased cells for both diagnostic and research purposes. In addition, engineered microparticle may be applied to molecular analyses including the isolation, separation, purification and identification of various materials such as proteins and nucleic acids. Further, techniques disclosed herein may be used in conjunction with current methods of separating cells, such as flow cell cytometry.

## BRIEF DESCRIPTION OF THE DRAWINGS

The following drawings form part of the present specification and are included to further demonstrate certain

EP 1 305 628 B1

aspects of the present invention. The invention may be better understood by reference to one or more of these drawings in combination with the detailed description of specific embodiments presented herein.

FIG. 1 shows a dielectrically-engineered microparticle according to one embodiment of the present disclosure.

FIG. 2A and FIG. 2B are dielectrophoretic force diagrams. The diagrams show the dielectrophoretic force vectors experienced by a spherical particle of radius 5 $\mu$m in a rotating field produced by phase quadrature voltage signals of 1 $V_{rms}$ applied to the electrodes. In FIG. 2A, Re($f_{CM}$) = 0.5 and Im($f_{CM}$) = 0. In FIG. 2A, the force directs particles towards the electrode located along the edges of the figures. In FIG. 2B Re($f_{CM}$) = 0 and Im($f_{CM}$) = 0.5. The forces in FIG. 2B direct particle circular translation about the center of the electrode geometry.

FIG. 3 shows a graph of electrorotation spectra. In particular, typical ROT spectra for erythrocytes ($\Delta$), T-lymphocytes (O) and MDA231 breast cancer cells ($\square$) in isotonic sucrose of conductivity 56 mS/m are shown. Curves show best fits of a single-shell dielectric model, discussed below.

FIG. 4 shows a graph of AC electrokinetic behavior of different microparticle types. The cDEP (conventional Dielectrophoresis) and twDEP (travelling wave DEP) response for five different microparticle types are shown. Each microparticle type is identical except for the thickness of their outermost shells, which vary from about 1 - 10 nm. Each different type of microparticle may be linked to a different probe and used to label and then manipulate or identify different analytes in a sample mixture.

FIG. 5 is a schematic diagram showing the separation of analytes. Three different types of engineered microparticles according to one embodiment of the present disclosure (denoted *a, b* and *c*) with AC electrokinetic properties such as those illustrated in FIG. 4, are sensitized with antibodies for CD3, CD4 and CD18 cell surface antigens to form labels for different cell subpopulations. These labels facilitate DEP-FFF (DEP/ field flow fractionation) separation of CD3$^+$, CD4$^+$ and CD18$^+$ cells as shown in the simulated DEP-FFF fractogram.

FIG. 6 is a schematic diagram showing the detection of analyte binding. The dielectric properties of engineered microparticles may be perturbed by analyte binding. An AC electrokinetic analysis method such as DEP-FFF may be used to detect this change in the form of elution peak broadening or elution peak shifting.

FIG. 7 is a graph showing the dependence of particle velocity on dielectric properties.

FIG. 8 is a schematic illustrating particle and medium polarization.

FIGS. 9A-15B show dielectrically-engineered microparticles, their properties, and behavior according to one embodiment of the present disclosure.

FIG. 16 is a schematic illustrating sandwich (double label) assays that may be used for detecting protein and mRNA in studies in accordance with the present disclosure.

FIG. 17 shows a dielectrically-engineered microparticle according to one embodiment of the present disclosure including a polystyrene core, a gold shell, and an alkanethiol self-assembled monolayer.

FIG. 18 shows a dielectrically-engineered microparticle according to one embodiment of the present disclosure including a polystyrene core, a gold shell, an alkanethiol self-assembled monolayer, and a phospholipid self-assembled monolayer.

FIG. 19 shows a dielectrically-engineered microparticle according to one embodiment of the present disclosure including a polystyrene core, a gold shell, an alkanethiol self-assembled monolayer, and a cross-linked phospholipid self-assembled monolayer.

FIG. 20 shows a dielectrically-engineered microparticle according to one embodiment of the present disclosure including a polystyrene core, a gold shell, an alkanethiol self-assembled monolayer, a phospholipid self-assembled monolayer, and a nucleic acid probe.

FIG. 21 shows a dielectrically-engineered microparticle according to one embodiment of the present disclosure including a polystyrene core, a gold shell, an alkanethiol self-assembled monolayer, a phospholipid self-assembled monolayer, and a protein probe.

FIG. 22 is a graph illustrating crossover frequency versus conductivity.

## DESCRIPTION OF ILLUSTRATIVE EMBODIMENTS

This disclosure describes a new technology in which microparticles are designed and produced with certain predetermined, or *engineered,* dielectric and/or magnetic properties such that their AC electrokinetic (including conventional dielectrophoresis (cDEP), traveling wave dielectrophoresis (twDEP), traveling wave dielectrophoresis (gDEP) or electrorotation (ROT)) and magnetophoretic (MAP) behavior is at least partially calculable or controllable.

These engineered-microparticles may be sensitized with various probes such as antibodies, nucleic acids or chemical ligands by methods known in the art and correspondingly used to label a variety of different analyte types including, but not limited to, cells, subcellular components, and biomolecules. Analytes labeled with such engineered microparticles may then be manipulated using existing AC electrokinetic or magnetophoretic methods (or a combination thereof).

Since different classes of engineered microparticles may be designed with different AC electrokinetic and/or magnetophoretic responses, several different analytes in a mixture can simultaneously be labeled with one or more probes and then individually (or as a group defined by each type of different probe) addressed, manipulated and/or identified. This ability may be referred to as indexing and represents a significant advance over existing technology. Further, as new AC electrokinetic and magnetophoretic analysis methods are developed, the engineered microparticle labels discussed herein may be used with those methods to address, manipulate, and identify analytes.

In addition, previously disclosed AC electrokinetic analysis methods such as dielectrophoretic field-flow fractionation (DEP-FFF), travelling-wave DEP (twDEP), and spiral electrode methods may be enhanced through the use of the engineered microparticle labels discussed herein. These previously disclosed methods typically exploit differences in the *inherent* dielectric properties of analytes to achieve analyte manipulation and identification. Probe-sensitized engineered-microparticles, on the other hand, provide techniques for separating analytes with *unknown* or *indiscriminable* dielectric properties and for identifying analytes by sensing changes in engineered microparticle behavior following analyte binding. Engineered dielectric and magnetic microparticle labels are therefore an enabling technology that may provide powerful new methods for separating and identifying analytes in many diverse fields.

Several types of engineered microparticles may be used to simultaneously label and probe a sample for multiple target analytes. Because each microparticle type may be engineered to have a specific, distinguishable dielectric and/or magnetic response, different target analytes in the mixture may be independently manipulated, sequentially or in parallel.

Additionally, engineered microparticles according to the present disclosure, may be sensed, and hence identified, by methodology known in the art.

Additionally, engineered microparticles may be discriminated, sorted and processed by AC electrokinetic and/or magnetophoretic methods. Because the discrimination of some AC electrokinetic based methods is orders of magnitude better than existing isolation methods, is controllable by electronic means, and unlike existing methods, is applicable to integrated and automated microsystems for chemical and biological analysis, the engineered microparticles of this disclosure may be used to solve many, if not all, of the shortcomings addressed above in relation to the existing technology in this field.

Different engineered microparticle types, each designed with unique intrinsic dielectric properties, may be indexed based upon their dielectric properties and used as frequency dependent *dielectric handles* to manipulate different analytes simultaneously. A library of engineered microparticles may then be developed with different dielectric properties. Such a library may be used to perform indexing. The library may also be used to develop bead-based biochemical assays for several different types of applications such as for microflumes.

In one embodiment, analytes may be detected using a *sandwich* protocol. In such a system, a change in bead fluorescence is the result of two separate binding events, mediated by the presence of a specific analyte. Engineered microparticles may first be sensitized (or linked) with a *capture probe* that has high binding affinity for a specific analyte. These sensitized engineered microparticles may then incubated with a sample droplet, resulting in the formation of engineered microparticle-analyte complexes. A droplet containing a *labeling probe* with high affinity for a secondary epitope or nucleic acid sequence on the analyte may then added to the reaction mixture, resulting in the formation of engineered microparticle-analyte-flurophore complexes. These complexes may be pulled to a reaction surface using positive dielectrophoresis and held *in situ* while the suspending droplet is pulled away. A different reagent droplet may then be moved over the engineered microparticle complexes and the DEP force removed. The engineered microparticles may be spontaneously released into and thennokinetically mixed with the new reagent, resulting in a buffer change or washing operation. This ability to reversibly immobilize analytes in a microfluidic device without the use of a probe that is permanently linked to the surface of the device represents a major advance in microflume-based chemical analysis.

Calibration, sample carryover, and cross-contamination problems known in the art may be addressed by using molecular recognition and sensing elements that are attached to engineered microparticles so that a new aliquot of sensitized engineered microparticles can be used for each and every assay. By disposing of the microparticles afterwards, by running a "blank" between each sample, and by allowing for cleaning cycles, calibration issues may be addressed and the absence of carryover and cross-contamination can be verified. Placing biologically active components on engineered microparticles also means that a single fluidic device may be applied to a wide range of sample preparation and molecular analysis problems by using different engineered microparticle/probe combinations. Finally, because no biological components need to be attached to fixed surfaces those surfaces may be PTFE coated, for example, to reduce biomolecular adhesion and carryover issues. It follows that the decision to use the engineered microparticles of the present disclosure may enhance the potential applicability of the technology by allowing a single device to have multiple applications.

Fabricating engineered microparticles according to the present disclosure creates the opportunity to conduct molecular analyses in parallel using a cocktail of different engineered microparticle/probe combinations. Assays using engineered beads require minimal quantities of sample. For example, a engineered microparticle of 5 $\mu$m diameter has the relatively large surface area of approximately 78 $\mu$m$^2$ yet occupies a volume of only 65 fL, about 1/15 that of a typical tumor cell. 100 tumor cells and 250 engineered microparticles comprised of 10 different engineered microparticle types may be packed into spherical region of 50 µm diameter using DEP-mediated focusing. This is the equivalent of almost $10^9$ cells/ml held in contact with 2 x $10^9$ engineered microparticles/ml carrying the molecular probes. The time for hybridization of target mRNA's to cDNA probes on magnetic microparticle surfaces has been shown to be just a few minutes in concentrated cell lysates. Therefore, the engineered microparticle-based approach described herein, using such high cell and engineered microparticle concentrations, has the potential to enable rapid assays for molecular markers in an integrated system.

In developing engineered microparticle-based indexing technology of the present disclosure, a reduced panel of 10 or so key molecular markers may be selected from a library of available markers for the purpose of screening for specific subsets of suspected disease states. By combining sample preparation and molecular analysis into a single, automated process, this system allows for the exploitation of gene-chip-derived molecular epidemiological data and render it accessible to a wide population.

**Engineered Microparticles**

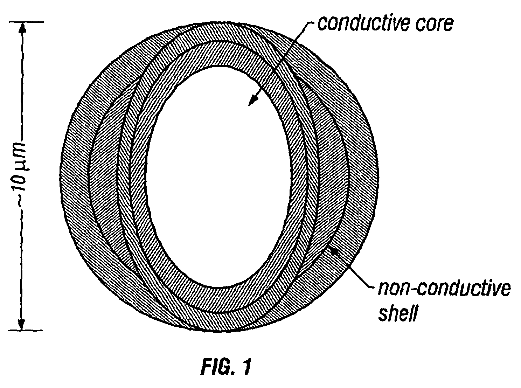

The structure of an engineered microparticle according to one embodiment of the present disclosure is shown in FIG. 1. There, a conductive core surrounded by a thin, poorly conducting, dielectric shell is illustrated. The conductive core may be made of a wide variety of materials. Further, the conducting core may be solid or hollow. Still further, the conducting core may be formed from an insulating inner region surrounded in whole, or in part, by a conducting outer region.

Although the shape of the inner core may vary somewhat, the shape may be spherical in one embodiment. In other embodiments, however, the shape may be elliptical or any other suitable shape.

The dielectric shell may be formed from a number of materials suitable to create desired dielectric properties, and specifically, properties that will provide for the dielectrophoretic responses at given frequencies. The dielectric shell may be coupled to the inner conductive core by any manner known in the art.

The shape of the outer dielectric shell may vary as well, but in one embodiment, it may generally be spherical or, generally, conform to the shape of the inner conductive core.

The size, composition, thickness, and shape of the conductive core and/or the dielectric shell may all be adjusted and optimized so as to achieve desired dielectric and/or magnetic properties. In particular, the sizes, thicknesses and compositions may be adjusted so that an engineered microparticle has the proper dielectric properties to be manipulated by a certain range of dielectrophoretic forces.

In one embodiment, polystyrene-coated silver microparticles may be used as engineered microparticles. These engineered microparticles undergo a frequency-dependent change from a non-conducting state to a conducting state. This is the result of a dielectric dispersion in which an AC field of appropriate frequency penetrates through the non-conducting polystyrene shell.

In another embodiment, a fabrication process using self-assembled monolayers (SAMs) of alkanethiolate on gold or silver-coated, hollow glass (or polystyrene or other microparticle) microparticles may be used to produce improved biomimetic particles. The dielectrophoretic behavior of these engineered microparticles may be predicted using established dielectrophoretic and multi-shell models known in the art, and the effects of changing engineered microparticle properties such as particle diameter and insulating layer thickness and composition may be determined by methods known in the art.

The engineered microparticle of FIG. 1 may be designed to mimic a mammalian cell. Specifically, it may be engineered so that its AC electrokinetic behavior mimics that of mammalian cells. This behavior has been characterized extensively for cells and is distinguished by a well-defined and relatively sharp frequency dependence known in the art. Samples of these microparticles have been produced by encapsulating conductive core particles of silver with non-conducting shells of various thicknesses of polystyrene. The cDEP response of these particles has been studied and shown to vary in accordance with the predictions of established AC electrokinetic theory.

Classes of engineered microparticles different than that illustrated in FIG. 1 may be designed and produced according to manufacturing principles known in the art. Again, within each structural class of microparticles, a range of different dielectric responses may be achieved by varying the compositions, thicknesses, and/or other properties of the layers comprising the individual microparticles. In this way, a library of engineered microparticles having well defined, yet clearly distinguishable, dielectric and/or magnetic properties may be produced. By designing and fabricating different microparticle types with distinct dielectric and/or magnetic properties, each type of engineered microparticle may be independently addressed, manipulated, and characterized even when it is part of a mixture of multiple types of engineered microparticles.

### Linking Elements

According to one embodiment, the engineered microparticles discussed above may be coupled to one or more linking elements, or probes, in order to act as engineered microparticle labels. The general use of different linking elements is known in the art. However, sections below explain several specific embodiments relating to different linking elements that may be used in conjunction with the engineered microparticles described above. Those having skill in the art, having the benefit of the present disclosure, will recognize that other linking elements may, however, be used.

The term "linking element" or "probe" as used herein refers to any component that has an affinity for another component termed here as a "target analyte." The binding of the linking element to the target analyte forms an affinity pair between the two components.

For example, such affinity pairs include, for instance, biotin with avidin/streptavidin, antigens or haptens with antibodies, heavy metal derivatives with thiogroups, various polynucleotides such as homopolynucleotides as poly dG with poly dC, poly dA with poly dT and poly dA with poly U. Any component pairs with strong affinity for each other can be used as the affinity pair. Suitable affinity pairs are also found among ligands and conjugates used in immunological methods.

The choice of linking element will obviously depend on the nature of the microparticle and the target analyte. For instance, if one wishes to capture a nucleic acid species (the target analyte) on a microparticle, the linking element will normally be chosen to be a nucleic acid or nucleic acid analogue oligomer having a sequence complementary to that of the target analyte or a part thereof.

The linking element may be bound first to the microparticle and may then be a species having an affinity for the target analyte. Preferably, the affinity for the target analyte is a selective affinity such that the formation of the complex between the microparticle and the target analyte is selective and provides at least a degree of identification of the target analyte. Preferably, the affinity is highly specific and accordingly the linking element bound to the particle, which provides the selective affinity for the target analyte, may be an antibody or an antibody fragment having antibody activity, an antigen, a nucleic acid probe or a nucleic acid analogue probe having selective affinity for complementary nucleic acid sequences, or avidin or an avidin-like molecule such as strept-avidin.

### Nucleic Acids as Linking Elements

Nucleic acid based linking elements may be synthetic oligonucleotides, single-stranded DNA, complimentary DNA (cDNA), and RNA. Although shorter oligonucleotides may be easier to make, numerous other factors are involved in determining the specificity of hybridization. Both binding affinity and sequence specificity of an oligonucleotide to its complementary target increases with increasing length. It is contemplated that exemplary oligonucleotides of 8, 9, 10, 11, 12, 13, 14, 15, 16, 17, 18, 19, 20, 25, 30, 35, 40, 45, 50, 55, 60, 65, 70, 75, 80, 85, 90, 95, 100 or more base pairs will be used, although others are contemplated. Longer polynucleotides encoding 250, 300, 350, 400, 450, 500, 550, 600, 650, 700, 750, 800, 850, 900, 950, 1000, 1100, 1150, 1200, 1250, 1300, 1500, 1922, 2000, 3000, 4000 bases and longer are contemplated as well.

### Antibodies as Linking Elements

Antibody based linking elements refers to monoclonal or polyclonal antibodies, single chain antibodies, or recombinantly engineered antibodies. As used herein, the term "antibody" is intended to refer broadly to any immunologic binding agent such as IgG, IgM, IgA, IgD and IgE. The term "antibody" is used to refer to any antibody-like molecule that has an antigen binding region, and includes antibody fragments such as Fab', Fab, $F(ab')_2$, single domain anti-bodies (DABs), Fv, scFv (single chain Fv), and the like. Generally, IgG and/or IgM are preferred because they are the most common antibodies in the physiological situation and because they are most easily made in a laboratory setting. Means for preparing and characterizing antibodies are well known in the art (*See, e.g.,* Antibodies: A Laboratory Manual, Cold Spring Harbor Laboratory, 1988, incorporated herein by reference).

Methods for generating polyclonal antibodies are well known in the art. Briefly, a polyclonal antibody is prepared by immunizing an animal with an antigenic composition and collecting antisera from that immunized animal. A wide range of animal species can be used for the production of antisera. Typically the animal used for production of antisera is a rabbit, a mouse, a rat, a hamster, a guinea pig or a goat. Because of the relatively large blood volume of rabbits, a rabbit is a preferred choice for production of polyclonal antibodies.

As is well known in the art, a given composition may vary in its immunogenicity. It is often necessary therefore to boost the host immune system, as may be achieved by coupling a peptide or polypeptide immunogen to a carrier. Exemplary and preferred carriers are keyhole limpet hemocyanin (KLH) and bovine serum albumin (BSA). Other albumins such as ovalbumin, mouse serum albumin or rabbit serum albumin can also be used as carriers. Means for conjugating a polypeptide to a carrier protein are well known in the art and include glutaraldehyde, m-maleimidobenzoyl-N-hydroxysuccinimide ester, carbodiimide and bis-biazotized benzidine.

As is also well known in the art, the immunogenicity of a particular immunogen composition can be enhanced by the use of non-specific stimulators of the immune response, known as adjuvants. Exemplary and preferred adjuvants include complete Freund's adjuvant (a non-specific stimulator of the immune response containing killed *Mycobacterium tuberculosis*), incomplete Freund's adjuvants and aluminum hydroxide adjuvant.

The amount of immunogen composition used in the production of polyclonal antibodies varies upon the nature of the immunogen as well as the animal used for immunization. A variety of routes can be used to administer the immunogen (subcutaneous, intramuscular, intradermal, intravenous and intraperitoneal). The production of polyclonal antibodies may be monitored by sampling blood of the immunized animal at various points following immunization. A second, booster injection, may also be given. The process of boosting and titering is repeated until a suitable titer is achieved. When a desired level of immunogenicity is obtained, the immunized animal can be bled and the serum isolated and stored, and/or in some cases the animal can be used to generate monoclonal antibodies (MAbs). For production of rabbit polyclonal antibodies, the animal can be bled through an ear vein or alternatively by cardiac puncture. The removed blood is allowed to coagulate and then centrifuged to separate serum components from whole cells and blood clots. The serum may be used as is for various applications or the desired antibody fraction may be purified by well-known methods, such as affinity chromatography using another antibody or a peptide bound to a solid matrix.

Monoclonal antibodies (MAbs) may be readily prepared through use of well-known techniques, such as those exemplified in U.S. Patent 4,196,265. Typically, this technique involves immunizing a suitable animal with a selected immunogen composition, *e.g.*, a purified or partially purified expressed protein, polypeptide or peptide. The immunizing composition is administered in a manner that effectively stimulates antibody producing cells.

The methods for generating monoclonal antibodies (MAbs) generally begin along the same lines as those for preparing polyclonal antibodies. Rodents such as mice and rats are preferred animals, however, the use of rabbit, sheep or frog cells is also possible. The use of rats may provide certain advantages (Goding, 1986, pp. 60-61), but mice are preferred, with the BALB/c mouse being most preferred as this is most routinely used and generally gives a higher percentage of stable fusions.

The animals are injected with antigen as described above. The antigen may be coupled to carrier molecules such as keyhole limpet hemocyanin if necessary. The antigen would typically be mixed with adjuvant, such as Freund's complete or incomplete adjuvant. Booster injections with the same antigen would occur at approximately two-week intervals.

Following immunization, somatic cells with the potential for producing antibodies, specifically B lymphocytes (B cells), are selected for use in the MAb generating protocol. These cells may be obtained from biopsied spleens, tonsils or lymph nodes, or from a peripheral blood sample. Spleen cells and peripheral blood cells are preferred, the former because they are a rich source of antibody-producing cells that are in the dividing plasmablast stage, and the latter because peripheral blood is easily accessible. Often, a panel of animals will have been immunized and the spleen of animal with the highest antibody titer will be removed and the spleen lymphocytes obtained by homogenizing the spleen with a syringe. Typically, a spleen from an immunized mouse contains approximately $5 \times 10^7$ to $2 \times 10^8$ lymphocytes.

The antibody-producing B lymphocytes from the immunized animal are then fused with cells of an immortal myeloma cell, generally one of the same species as the animal that was immunized. Myeloma cell lines suited for use in hybridoma-producing fusion procedures preferably are non-antibody-producing, have high fusion efficiency, and have enzyme deficiencies that render them incapable of growing in certain selective media that support the growth of only the desired fused cells (hybridomas).

Any one of a number of myeloma cells may be used, as are known to those of skill in the art (Goding, 1986). For example, where the immunized animal is a mouse, one may use P3-X63/Ag8, X63-Ag8.653, NS1/1.Ag 4 1, Sp210-Ag14, FO, NSO/U, MPC-11, MPC11-X45-GTG 1.7 and S194/5XX0 Bul; for rats, one may use R210.RCY3, Y3-Ag 1.2.3, IR983F and 4B210; and U-266, GM1500-GRG2, LICR-LON-HMy2 and UC729-6 are all useful in connection with human cell fusions.

One preferred murine myeloma cell is the NS-1 myeloma cell line (also termed P3-NS-1-Ag4-1), which is readily available from the NIGMS Human Genetic Mutant Cell Repository by requesting cell line repository number GM3573. Another mouse myeloma cell line that may be used is the 8-azaguanine-resistant mouse murine myeloma SP2/0 non-producer cell line.

Methods for generating hybrids of antibody-producing spleen or lymph node cells and myeloma cells usually comprise mixing somatic cells with myeloma cells in a 2:1 proportion, though the proportion may vary from about 20:1 to about 1:1, respectively, in the presence of an agent or agents (chemical or electrical) that promote the fusion of cell membranes. Fusion methods using Sendai virus have been described by Kohler and Milstein (1975; 1976), and those using polyethylene glycol (PEG), such as 37% (v/v) PEG, by Gefter *et al*. (1977). The use of electrically induced fusion methods is also appropriate.

Fusion procedures usually produce viable hybrids at low frequencies, about $1 \times 10^{-6}$ to $1 \times 10^{-8}$. However,

this low frequency does not pose a problem, as the viable, fused hybrids are differentiated from the parental, unfused cells (particularly the unfused myeloma cells that would normally continue to divide indefinitely) by culturing in a selective medium. The selective medium is generally one that contains an agent that blocks the *de novo* synthesis of nucleotides in the tissue culture media. Exemplary and preferred agents are aminopterin, methotrexate, and azaserine. Aminopterin and methotrexate block *de novo* synthesis of both purines and pyrimidines, whereas azaserine blocks only purine synthesis. Where aminopterin or methotrexate is used, the media is supplemented with hypoxanthine and thymidine as a source of nucleotides (HAT medium). Where azaserine is used, the media is supplemented with hypoxanthine.

The preferred selection medium is HAT. Only cells capable of operating nucleotide salvage pathways are able to survive in HAT medium. The myeloma cells are defective in key enzymes of the salvage pathway, *e.g.*, hypoxanthine phosphoribosyl transferase (HPRT), and thus they cannot survive. The B cells can operate this pathway, but they have a limited life span in culture and generally die within about two weeks. Therefore, the only cells that can survive in the selective media are those hybrids formed from myeloma and B cells.

This culturing provides a population of hybridomas from which specific hybridomas are selected. Typically, selection of hybridomas is performed by culturing the cells by single-clone dilution in microtiter plates, followed by testing the individual clonal supernatants (after about two to three weeks) for the desired reactivity. The assay should be sensitive, simple and rapid, such as radioimmunoassays, enzyme immunoassays, cytotoxicity assays, plaque assays, dot immunobinding assays, and the like.

The selected hybridomas would then be serially diluted and cloned into individual antibody-producing cell lines, which can then be propagated indefinitely to provide MAbs. The cell lines may be exploited for MAb production in two basic ways. A sample of the hybridoma can be injected (often into the peritoneal cavity) into a histocompatible animal of the type that was used to provide the somatic and myeloma cells for the original fusion. The injected animal develops tumors secreting the specific monoclonal antibody produced by the fused cell hybrid. The body fluids of the animal, such as serum or ascites fluid, can then be tapped to provide MAbs in high concentration. The individual cell lines could also be cultured *in vitro*, where the MAbs are naturally secreted into the culture medium from which they can be readily obtained in high concentrations. MAbs produced by either means may be further purified, if desired, using filtration, centrifugation and various chromatographic methods such as HPLC or affinity chromatography.

Large amounts of the monoclonal antibodies may also be obtained by multiplying hybridoma cells *in vivo.* Cell clones are injected into mammals that are histocompatible with the parent cells, *e.g.*, syngeneic mice, to cause growth of antibody-producing tumors. Optionally, the animals are primed with a hydrocarbon, especially oils such as pristane (tetramethylpentadecane) prior to injection.

In accordance with the present disclosure, fragments of the monoclonal antibody may be obtained from the monoclonal antibody produced as described above, by methods which include digestion with enzymes such as pepsin or papain and/or cleavage of disulfide bonds by chemical reduction. Alternatively, monoclonal antibody fragments encompassed by the present invention can be synthesized using an automated peptide synthesizer, or by expression of full-length gene or of gene fragments in *E. coli*.

**Other Linking Elements**

Other linking elements include peptides, antitumor agents, antibiotics and other therapeutic compounds. Again, what is required of these linking elements is the ability to bind with a degree of specificity to a target analyte. The use of peptides, antitumor agents, antibiotics and other therapeutic compounds would allow the ability to identify or purify such target analytes as receptors, cofactors, enzymes, or any other target capable of binding to the linking element.

Examples of the peptide linking elements include LH-RH antagonists (see U.S. Patents 4,086,219, 4,124,577, 4,253,997 and 4,317,815), insulin, somatostatin, somatostatin deruvatives (see U.S. Pat. Nos. 4,087,390, 4,093,574, 4,100,117 and 4,253,998), growth hormone, prolactin, adrenocorticotropic hormone (ACTH), melanocyte-stimulating hormone (MSH), thyrotropin-releasing hormone (TRH), their salts and derivatives (see JP-A 50-121273 and JP-A 52-116465), thyroid-stimulating hormone (TSH), luteinizing hormone (LH), follicle-stimulation hormone (FSH), vasopressin, vasopressin derivatives, oxytocin, calcitonin, parothyroid hormone, glucagon, gastrin, secretin, pancreozymin, cholecystokinin, angiotensin, human placental lactogen, human chorionic gonadotropin (HCG), enkephalin, enkephalin derivatives (see U.S. Patent 4,277,394 and EP-A 31,567); and polypeptides such as endorphin, kyotorphin, interferon ($\alpha$-type, $\beta$-type, $\gamma$-type), interleukin (I to XI), tuftsin, thymopoietin, tymosin, thymosthymlin, thymic hormone factor (THF), serum thymic factor (FTS) and derivatives thereof (see U.S. Patent 4,299,438), tumor necrosis factor TNF), colony stimulating factor (CSF), motilin, deinorphin, bombesin, neurotensin, caerulein, bradykinin, urokinase, asparaginase, kallikrein, substance P, nerve growth factor, blood coagulation factor VIII and IX, lysozyme chloride, polymyxin B, colistin, gramicidin, bacitracin, protein synthesis-stimulating peptide (see G.B. Patent No. 8,232,082), gastric inhibitory polypeptide (GIP), vasoactive intestinal polypeptide (VIP), platelet-derived growth factor (PDGH), growth hormone-releasing factor (GRF, somatoclinine), bone morphagenetic protein (BMP), epidermal growth hormone (EGF) and the like.

Examples of an antitumor agent include bleomycin hydrochloride, methotrexate, actinomycin D, mitomycin C, vinblastine sulfate, vincristine sulfate, daunorubicin hydrochloride, adriamynin, neocarzinostatin, cytosine arabinoside, fluorouracil, tetrahydrofuryl-5-fluorouracil, picibanil, lentinan, levamisole, bestatin, azimexon, glycyrrhizin, poly A: U, poly ICLC and the like.

Examples of an antibiotic include gentamicin, dibekacin, kanendomycin, lividomycin, tobromycin, amikacin, fradiomycin, sisomysin, tetracycline, oxytetracycline, roliteracycline, doxycycline, ampicillin, piperacillin, ticarcillin, cefalotin, cefaloridine, cefotiam, cefsulodin, cefinenoxime, cefmetazole, cefazollin, cefataxim, cefoperazone, ceftizoxime, moxolactame, thienamycin, sulfazecine, azusleonam, salts thereof, and the like.

Examples of therapeutic drugs include antipyretic, analgesic and antiinflammatory agents such as salicylic acid, sulpyrine, flufenamic acid, diclofenace, indometacin, morphine, pethidine, levorphanol tertrate, oxymorphone and the like; antitussive expectorants such as ephedrine, methylephedrine, noscapine, codeine, dihydrocodeine, alloclamide, chlorphezianol, picoperidamine, cloperastine, protokylol, isoproterenol, salbutamol, terebutaline, salts thereof and the like; sedatives such as chlorpromazine, prochloperazine, trifluoperazine, atropine, scopolamine, salts thereof and the like; muscle relaxant such as pridinol, tubocurarine, pancuronium and the like; antiepileptic agents such as phenytoin, ethosuximide, acetazolamide, chlordiazepoxide and the like; antiulcer agents such as metoclopramide, histidine and the like; antidepressant such as imipramine, clomipramine, onxiptiline, phenelzine and the like; antiallergic agent such as diphenhydramine hydrochloride, chlorpheniramine malate, tripelennamine hydrochloride, methdilazine hydrochloride, clemizole hydrochloride, diphenylpyraline hydrochloride, methoxyphenemine hydrochloride and the like; cardiotonics such as transpieoxocamphor, terephylol, aminophylline, etilifrine and the like; antiarrythmic agents such as propranolol, alprenolol, bufetololoxyprenolol and the like; vasodilators such as oxyfedrine, diltiazem, tolazoline, hexobendine, bamethan and the like; hypotensive diuretics such as hexamethonium bromide, pentolinium, mecamylamine, ecarazine, clonidine and the like; antidiabetic agents such as glymidine, glipizide, phenformin, buformin, metformin and the like; anticoagulants such as heparin, citric acid and the like; hemostatic agents such as thromboplastin, thrombin, menadione, acetomenaphthone, $\varepsilon$-aminocaproic acid, tranexamic acid, carbazochrome sulfonate, adrenochrome monoaminoguanidine and the like; antituberculous agents such as isoniazid, ethambutol, para-aminosalicylic acid and the like; hormones agents such as prednisolone, dexamethasone, betametasone, hexoestrol, methymazole and the like; narcotic antagonists such as levallorphan, nalorphine, naloxone, salts thereof and the like.

**Attaching Linking Elements to Engineered microparticles**

The nature of the method used to convert an original engineered microparticle into a recognition element by complexing it with a linking element can vary widely according to the nature of the linking element.

In some cases, the complex may involve a crosslinking agent connecting the engineered microparticle and the linking element. The complex may further include a label connected to the linking element or microparticle, optionally via a second linking element. The complex may involve numerous linking elements bound to the particle.

Antibodies and antibody fragments having antibody properties may be used for attachment. There are techniques suitable for coating antibodies on to the surface of microparticles which are well known to those skilled in the art. Antibody coated particles are capable of recognizing and binding corresponding antigens which may be presented on micro-organism cells or some other target analyte.

Methods are also known for binding oligo-nucleic acid probes to microparticles, such as the engineered microparticles described herein. Suitable techniques are by way of example described in Patent Application No. WO 93/04199. Where the linking element is a nucleic acid probe or a nucleic acid analogue probe, the resulting microparticle will of course be suitable for recognizing and binding complementary nucleic acid sequences.

Other methods of attaching linking elements to microparticles involve the use of functionalized crosslinking agents. Such crosslinking reagents are well known to those of skill in the art. A useful reference describing the scope of crosslinkers that are commonly available and their uses and limitations may be found in the Pierce Chemical Catalogue (Rockford, IL).

**Labels**

The use of an additional label to further increase the detectability of an engineered microparticle as well as to alter its magnetic and electrokinetic characteristics may be utilized. For instance, antibodies bearing fluorophores or chromaphores may be bound to an engineered microparticle so that the complex so-formed can be further distinguished from the starting engineered microparticle by magnetic and/or electrokinetic means as well as detection by fluorescence or color.

Such a label may be bound to the microparticle or linking element either before, simultaneously with, or after the formation of the complex between the target analyte and the engineered microparticle. The label may include a second linking element carried by the label. Once again, it is preferred that the affinity for the target analyte possessed by the second linking element is selective, preferably highly specific and the second linking element may also be an antibody, an antibody fragment having antibody activity, an antigen, a nucleic acid probe, a nucleic acid analogue probe, avidin or an avidin-like molecule. The use of a label of this nature may be desired to aid ready detection of the complex and/or where a complex between the microparticle and the target analyte does not in itself possess sufficiently distinctive magnetic and electrokinetic properties, thus the magnetic and electrokinetic may be further altered by the inclusion in the complex of the label. To this end, the label may be a fluorophore or chromaphore, or a micro-organism, a metal particle, a polymer bead or a magnetic particle. A suitable material is colloidal gold which is easily bound to antibodies (as the second species) to form a label. Antibodies bound to colloidal gold are commercially available and methods for binding antibodies to colloidal gold are for instance described in Geohegan et al. (1978). Other metal particles however may be employed, e.g. silver particles and iron particles.

The use of a label of the kind described above may be suitable even where a complex between the ligand and a particle possesses sufficiently distinctive magnetic and electrokinetic properties to enable the formation of such a complex to be observed. A higher level of specificity may in certain cases be obtained by the use of a label in such a complex. Thus for instance, one may wish to distinguish a microorganism expressing an antigen A from a micro-organism expressing antigens A and B. This may be accomplished by the use of engineered microparticles having as a linking element an antibody to A and a label having as it's linking element an antibody to B. The difference in the characteristics of the labeled complex (between the engineered microparticle, the microorganism and the label) and the unlabeled complex (between the engineered microparticle and the micro-organism) can be observed, and used to distinguish microorganisms expressing antigen A only, from those expressing A and B.

Labels for both cells and smaller particles can include fluorescent markers, e.g. FITC or rhodamine, chromophores, luminescent markers or enzyme molecules which can generate a detectable signal. Examples of the latter include luciferases and alkaline phosphatase. These markers may be detected using spectroscopic techniques well known to those skilled in the art.

**Exemplary Microparticle-based Labels and Their Uses**

The following engineered microparticle based labels are included to further delineate uses of the present disclosure. The following patent applications and publications describe linking elements, crosslinkers or methods of bonding or attaching linking elements to microparticles, target analytes, and other methodologies that may be employed in the present invention.

A method for determining the concentration of substances in biological fluids (e.g., drugs, hormones, vitamins and enzymes) wherein magnetically responsive, permeable, solid, water insoluble, microparticles are employed is disclosed in U.S. Patent 4,115,534.

U.S. Patent 4,285,819 describes microparticles which may be employed to remove dissolved ions from waste aqueous streams by formation of chelates. U.S. Patent 3,933,997 describes a solid phase radio immunoassay for digoxin where anti-digoxin anti-bodies are coupled to magnetically responsive particles.

Small magnetic particles coated with an antibody layer are used in U.S. Patent 3,970,518 to provide a large and widely distributed surface area for sorting out and separating select organisms and cells from populations thereof. U.S. Patent 4,018,886 discloses small magnetic particles used to provide a large and widely distributed surface area for separating a select protein from a solution to enable detection thereof. The particles are coated with a protein that will interact specifically with the select protein.

U.S. Patent 4,070,246 describes compositions comprising stable, water insoluble coatings on substrates to which biologically active proteins can be covalently coupled so that the resulting product has the biological properties of the protein and the mechanical properties of the substrate, for example, magnetic properties of a metal support.

A diagnostic method employing a mixture of normally separable protein-coated particles is discussed in U.S. Patent 4,115,535. Microparticles of acrolein homopolymers and copolymer(s) with hydrophilic comonomers such as methacrylic acid and/or hyroxyethylmethacrylate are discussed in U.S. Patent 4,413,070. U.S. Patent 4,452,774 discloses magnetic iron-dextran microparticles which can be covalently bonded to antibodies, enzymes and other biological molecules and used to label and separate cells and other biological particles and molecules by means of a magnetic field. Coated magnetizable microparticles, reversible suspensions thereof, and processes relating thereto are disclosed in U.S. Patent 4,454,234. A method of separating cationic from anionic beads in mixed resin beds employing a ferromagnetic material intricately incorporated with each of the ionic beads is described in U.S. Patent 4,523,996. A magnetic separation method utilizing a colloid of magnetic particles is discussed in U.S. Patent 4,526,681. U.K. Patent Application GB No. 2,152,664A discloses magnetic assay reagents.

An electron-dense antibody conjugate made by the covalent bonding of an iron-dextran particle to an antibody molecule is reported by Dutton et al. (1979). Ithakissios et al. (1977) describes the use of protein containing magnetic microparticles in radioassays. The separation of cells labeled with immunospecific iron dextran microparticles using high gradient magnetic chromatography is disclosed by Milday *et al.* (1984). Molday *et al.* (1982) describe an immunospecific ferromagnetic iron-dextran reagent for the labeling and magnetic separation of cells. An application of magnetic microparticles in labeling and separation of cells is also disclosed by Molday *et al.* (1977). A solid phase fluoroimmunoassay of human albumin and biological fluids is discussed by Margessi *et al.* (1978). Nye *et al.* (1976) disclose a solid phase magnetic particle radioimmunoassay. Magnetic fluids are described by Rosenweig (1983). Magnetic protein A microparticles and their use in a method for cell separation are disclosed by Widder *et al.* (1979).

U.S. Patent 5,279,936 is a method directed to the separation of a component of interest from other components of a mixture by causing the binding of the component of interest to magnetic particles. In the embodiment of the invention which is a method to separate cells from a mixture containing other components, the method comprises layering a first liquid medium containing cells and other components with a second medium which is of a different density than and/ or different viscosity than the first liquid medium. The cells are bound to paramagnetic particles. The layered first liquid medium and the second liquid medium are subjected to a magnetic field gradient to cause the cell particles to migrate into the second medium. The purpose of isolating the cells in the second liquid medium is then, by a further embodiment, to separate the cells from the second liquid medium.

U.S. Patent 4,935,147 is a method that specifically targets the application of magnetic separation in the assay of organic and inorganic biochemical analytes, particularly those analytes of interest in the analysis of body fluids. The method of this invention provides a way of separating non-magnetic particles from a medium by virtue of the chemically controlled non-specific reversible binding of such particles to magnetic particles. Because of the small size of the magnetic particles, it also provides for a very rapid binding of a substance to be separated. By then aggregating the particles there is provided a much more rapid and complete magnetic separation than has been achieved by previous methods.

Other current practices in the field for cell separation utilize matrix materials of, for example, hollow fibers, flat sheet membrane, or packed-bed bead or particle materials with physically adsorbed or covalently attached chemicals or antibodies for selective cell separation. These devices are designed to allow continuous whole blood or blood component inflow and return. Since these devices operate at normal blood flow rates under conditions in which the concentration of desired cells can be very low compared with other cell types, the separation process is often not efficient. Moreover, with these systems it is difficult to collect the selected cells in a viable state.

The development of paramagnetic beads offered the prospect of magnetic separation of target cells. Various methods to produce magnetic and paramagnetic particles are disclosed in the following United States patents: U.S. Patent 4,672,040; U.S. Patent 5,091,206; U.S. Patent 4,177,253; U.S. Patent 4,454,234; U.S. Patent 4,582,622; U.S. Patent 4,452,773; U.S. Patent 5,076,950; U.S. Patent 4,554,088; and U.S. Patent 4,695,392.

Various methods were devised to use magnetic particles for assays. See, for example, United States patents: U.S. Patent 4,272,510; U.S. Patent 4,777,145; U.S. Patent 5,158,871; U.S. Patent 4,628,037; U.S. Patent 4,751,053; U.S. Patent 4,988,618; U.S. Patent 5,183,638; U.S. Patent 4,018,886; and U.S. Patent 4,141,687.

Attempts were made to use magnetic particles for separation of biological components, including cells. The following is a list of United States patents known to the Applicants and believed to be directed to magnetic separators and methods: U.S. Patent 4,855,045; U.S. Patent 4,664,796; U.S. Patent 4,190,524; U.S. Patent 4,738,773; U.S. Patent 4,941,969; U.S. Patent 5,053,344; U.S. Patent 5,200,084; U.S. Patent 4,375,407 ; U.S. Patent 5,076,914; U.S. Patent 4,595,494; U.S. Patent 4,290,528; U.S. Patent 4,921,597; U.S. Patent 5,108,933; U.S. Patent 4,219,411; U.S. Patent 3,970,518; and U.S. Patent 4,230,685.

A number of techniques have been developed recently using microparticle-based methods to meet the demands for rapid and accurate detection of agents, such as viruses, bacteria and fungi, and detection of normal and abnormal genes. Such techniques, which generally involve the amplification and detection (and subsequent measurement) of minute amounts of target nucleic acids (either DNA or RNA) in a test sample, include inter alia the polymerase chain reaction (PCR) (Saiki *et al.*, 1985; 1988; PCR Technology, Henry A. Erlich, ed., Stockton Press, 1989; Patterson *et al.*, 1993), ligase chain reaction (LCR) (Barany, 1991), strand displacement amplification (SDA) (Walker *et al.*, 1992), Qβ replicase amplification (QβRA) (Wu *et al.*, 1992; Lomeli *et al.*, 1989) and self-sustained replication (3SR) (Guatelli *et al.*, 1990).

Other applications for such techniques include detection and characterization of single gene genetic disorders in individuals and in populations (see, e.g., Landergren *et al.*, 1988 which discloses a ligation technique for detecting single gene defects, including point mutations). Such techniques should be capable of clearly distinguishing single nucleotide differences (point mutations) that can result in disease (e.g., sickle cell anemia) as well as deleted or duplicated genetic sequences (e.g., thalassemia).

**Handles**

If different types of engineered microparticle are linked to different probes that are directed against specific analytes, different target analytes may be simultaneously labeled yet independently manipulated within an analyte mixture. Upon binding to its target analyte, a sensitized engineered microparticle label (an engineered microparticle coupled to one or more linking elements or additional labels) acts as a *handle* that may be used to pull the analyte from cell lysate, serum or other biological sample, for example. Numerous labeled analytes may simultaneously be manipulated in a switchable, frequency dependent manner.

In addition to acting as handles, probe-sensitized engineered microparticles may also be used for *detection* and, in one embodiment, simultaneously, or near-simultaneous detection of analytes. With the benefit of this disclosure, engineered microparticles may be designed such that the dielectric properties and, thus, the dielectrophoretic behavior are very sensitive to analyte binding. The presence of a target analyte in a sample may be detected by observing this change in AC electrokinetic behavior.

In light of the above, it is apparent to those skilled in the art that the engineered microparticles of the present disclosure, which may be used for AC electrokinetic manipulation of cells and biomolecules, provide enabling technology for the development of improved separation and detection methods for integrated and automated microsystems, where conventional methods such as centrifugation or immunodetection are difficult or impractical to implement. Devices utilizing these improved methods may be useful in a variety of diagnostic and research applications, as discussed earlier.

In one embodiment, the isolation, identification, etc. of suspect cells from mixed cell suspensions and the manipulation of mixtures of dielectrically indexed engineered microparticles may be achieved, all in an integrated device. Achieving these steps ultimately depends upon ways of moving matter with respect to the solution that suspends it, a problem to which dielectrophoresis is ideally suited. Although principles of dielectrophoresis are known in the art, sections below explain, and apply, certain of those principles to the engineered microparticles discussed herein.

**AC Electrokinetic Phenomena**

AC electrokinetic phenomena are a family of related effects in which alternating electric fields induce forces on particles. These forces depend upon the dielectric characteristics of particles and their surroundings. The best-known electrokinetic phenomenon is conventional dielectrophoresis (cDEP). The term *dielectrophoresis* (*DEP*) was first used by Pohl to describe the motion of polarizable particles towards the minimum of dielectric potential in a non-uniform electric field (Pohl, 1978; Sauer, 1985; Kaler and Jones, 1990; Holzel *et al*., 1991; Gascoyne *et al*., 1993). This phenomenon is exploited in cell fusion and electroporation devices (Abidor *et al*., 1994; Wu *et al*., 1994) in order to bring cells into close contact through *pearl chain* formation. More recently, other electrokinetic phenomena including electrorotation (ROT, particle rotation resulting from the torque exerted on the particle by a rotating electrical field) (Arnold and Zimmermann, 1982 and 1988; Fuhr *et al*., 1990; Hu et al, 1990; Gimsa *et al*., 1991; Holzel and Lamprecht, 1992; Huang *et al*., 1992; Sukhorukov *et al*., 1993; Wang *et al*., 1994c) and travelling-wave dielectrophoresis (twDEP, lateral motion of a particle caused by an electrical field sweeping through space) (Masuda *et al*., 1988; Hagedorn *et al*., 1992; Huang *et al*., 1993 Gascoyne *et al*., 1994a) have been investigated for their applicability in the noninvasive characterization and manipulation of cells and biomolecules.

AC electrokinetic phenomena result from the interaction between an electric field and polarizations induced in a particle by the field. The effect has been studied in detail by several groups, and its theory is fairly well established (Pething, 1979; Jones, 1995). It is important to note that while dielectrophoresis and the more familiar *electrophoresis* both describe electrokinetic phenomena, they are distinguished by several fundamental differences. In dielectrophoresis particle motion is determined by the magnitude, polarity, and phase of charges that are induced in a particle by an applied field. It is not necessary that the particle carry an intrinsic net charge to experience dielectrophoresis. Electrophoresis, however, requires that a particle carry an intrinsic net charge. It is the interaction between this intrinsic charge and an electric field that causes particle motion. Furthermore, electrophoresis typically utilizes homogeneous, direct current electric fields. Dielectrophoresis requires the use of inhomogeneous electric fields that can be either direct or alternating current. The AC electrokinetic phenomena, cDEP, twDEP, gDEP and ROT, have been considered very little for separation and analysis in chemistry and the life sciences, despite the fact that they are, by their very nature, far more versatile than the commonly used method of electrophoresis. Consider the following advantages of AC electrokinetic methods:

(1) The magnitude and sign of the charges induced in a particle depend strongly on particle dielectric properties. In the case of engineered microparticles, this includes particle coating and core material properties. Particles with a wide range of dielectric properties can be made by changing the thickness and composition of the coating as well as the composition of the core particle.

(2) The magnitude and sign of the charges induced in a particle are not fixed but depend critically on the frequency of the applied field and on properties of the medium the particle is suspended in. For this reason, the engineered microparticles may be individually addressed in a frequency dependent manner.

(3) AC electrokinetic phenomena embody not just one type of linear motion but a variety of kinetic effects in two dimensions that can be exploited not only to manipulate particles but also to characterize their dielectric properties.

(4) As discussed in (1) and (2), the AC electrokinetic response of a particle is highly sensitive to the dielectric properties of the particle. Engineered microparticles may be produced such that their dielectric properties are very sensitive to binding of analyte. Such engineered microparticles provide a means of discriminating unbound engineered microparticles from analyte-microparticle complexes. This type of engineered microparticle may be used for qualitative, and in some cases quantitative, identification of an analyte.

(5) The strong dependencies of cell motions in two dimensions on the field configuration, the field frequency and the suspending medium dielectric properties promise versatility of particle separation technologies targeted at a variety of different applications.

All of these phenomena may be used individually or simultaneously to exploit fully the dielectric properties of the particles by appropriate applied field configurations.

**Generalized Dielectrophoresis Theory**

The dielectrophoretic force acting on a particle due to an imposed electrical field vector $\vec{E}(t)$ can be written quite generally in terms of the effective dipole moment vector $\vec{m}(t)$ that the field induces in the particle (Huang *et al.*, 1992 and 1993; Gascoyne *et al.*, 1994b; Wang *et al.*, 1994a) as

$$\vec{F}(t) = (\vec{m}(t) \cdot \nabla)\vec{E}(t). \tag{1}$$

In the frequency domain, the induced particle dipole moment is given by

$$\vec{m}(\omega) = 4\pi \, \varepsilon_m \, r^3 \, f_{CM} \, \vec{E}(\omega) \tag{2}$$

where $\omega$ is the angular frequency of the applied field, r the particle radius, and $f_{CM}$ the Clausius-Mossotti factor defined as

$$f_{CM}(\varepsilon_p^*, \varepsilon_m^*) = \frac{\varepsilon_p^* - \varepsilon_m^*}{\varepsilon_p^* + 2\varepsilon_m^*} \tag{3}$$

Here $\varepsilon_p^*$ and $\varepsilon_m^*$ are the complex permittivities of the particle and its suspending medium, respectively. Until recently, expressions describing different electrokinetic phenomena including cDEP and twDEP (Huang *et al.*, 1993) were derived by substituting appropriate spatial expressions for $\vec{E}$, resulting in special cases of the force expression. However, by utilizing the fact that the mixed partial derivatives of the field with respect to space and time must obey the Swartz relationships (Gellert *et al.*, 1977) in order that the field remain continuous, it has recently been derived (Wang *et al.*, 1994a and 1994b) that the time-averaged dielectrophoretic force, may be represented as

$$\left\langle \vec{F}(t) \right\rangle = 2\pi\varepsilon_m \, r^3 \left( \mathrm{Re}(f_{CM}) \nabla E(rms)^2 + \mathrm{Im}(f_{CM}) \left( E_{x0}^2 \nabla \varphi_x + E_{y0}^2 \nabla \varphi_y + E_{z0}^2 \nabla \varphi_z \right) \right),$$

$$\tag{4}$$

where *E(rms)* is the rms value of the electric field strength. $E_{i0}$ and $\varphi_i$ (i=x; y; z) are the magnitude and phase, respectively, of the field components in the principal axis directions. Unlike previous analyses, this expression can be used to investigate the forces arising from any form of applied field. It contains two terms that allow an appreciation, for the first time, that there are two independent force contributions to gDEP motion:

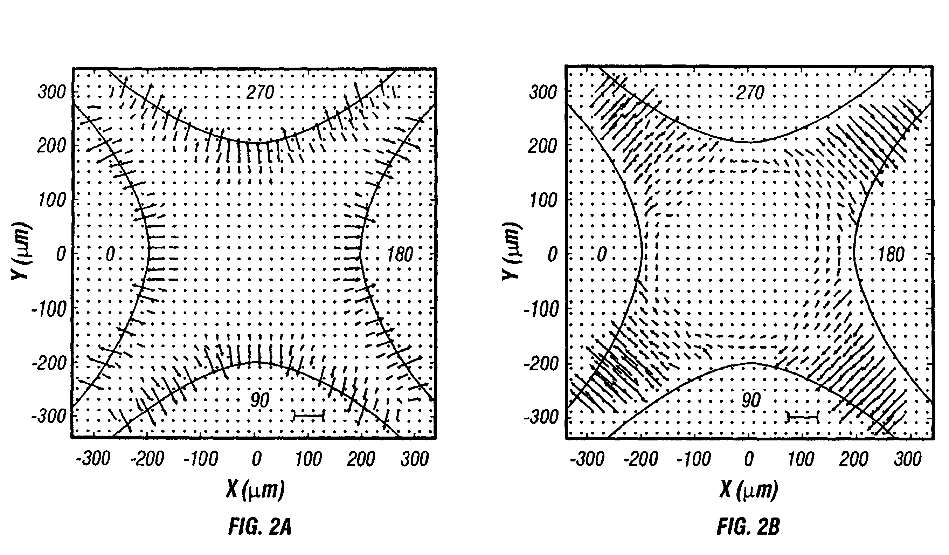

(i) the left hand term relates to the *real* (in-phase) component (Re($f_{CM}$)) of the induced dipole moment in the particle and to the spatial nonuniformity, $\nabla E(rms)^2$, of the field *magnitude*. This force directs the particle towards the strong or the weak field regions, depending upon whether Re($f_{CM}$) is positive or negative (FIG. 2a). This is the conventional DEP term (Huang *et al.*, 1992; Jones and Kallio, 1979).

(ii) the right hand term relates to the *imaginary* (out-of-phase) component of the induced dipole moment and to the field spatial nonuniformity ($\nabla\varphi_x$, $\nabla\varphi_y$ and $\nabla\varphi_z$) of the field *phase*. Depending on the polarity of Im($f_{CM}$), this force directs the particle towards regions where the phases of the field component are larger (Im($f_{CM}$) > 0) or smaller (Im($f_{CM}$) < 0) (FIG. 2b). Under the constraint conditions for a travelling electric field, Eq. 4 is reduced to the twDEP force expression (Huang et al, 1993).

Eq. 4 shows that the force experienced by a particle in an AC electric field arises not only from the field *magnitude* inhomogeneity as envisioned by Pohl (1978) but also from the field *phase* nonuniformity. The inventors have termed the particle motion caused by both magnitude and phase nonuniformities *generalized dielectrophoresis*. Since all field-induced cell motions are understandable in terms of Eq. 4, sophisticated field configurations having both phase and magnitude nonuniformities can be explored by methodology known in the art.

**Characterization Of Engineered Microparticles By Rot**

The dielectric properties of particles, including engineered microparticles, may be established by electrorotation. In electrorotation, particles are subjected to a rotating electric field and induced to rotate about an essentially stable axis. The method is suitable over other characterization methods as it is relatively straightforward, offers good reproducibility, and provides a means to characterize individual particles. While particle dielectric properties can be probed non-invasively by any of the electrokinetic phenomena, ROT offers the significant advantage that it induces particle rotation about an axis that, for most purposes, can be considered stationary in space. Thus, the particle remains in a position of constant field strength. The ROT torque, $\vec{\Gamma}(t)$, depends (Arnold and Zimmermann, 1982 and 1988; Fuhr, 1985) not on the inhomogeneity of the electrical field, but on the cross product

$$\vec{\Gamma}(t) = -\vec{m}(t) \times \vec{E}(t), \tag{5}$$

and in the frequency domain the magnitude of this torque (Arnold and Zimmermann, 1982 and 1988; Fuhr, 1985) can be written

$$\Gamma(\omega) = -4\pi\,\varepsilon_m\,r^3\,\text{Im}(f_{CM})E^2. \tag{6}$$

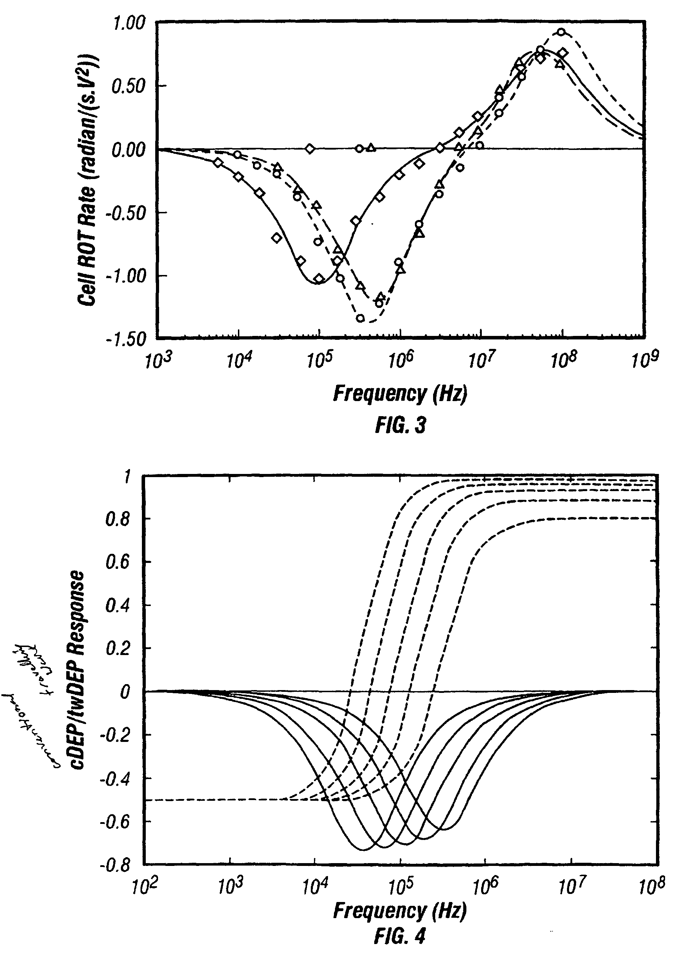

Typical ROT spectra derived for three different particle, or in this case cell, types are shown in FIG. 3. Equation 6 shows that the shape of each spectrum reflects Im($f_{CM}$) for each particle type. By applying an appropriate dielectric model, it is possible to derive the dielectric properties of engineered microparticles directly from their ROT spectra. Explicit dielectric modeling of particle properties is most frequently undertaken using dielectric shell models (Huang *et al.*, 1992; Fuhr, 1985; Irimajri *et al.*, 1979), and the inventors (Huang *et al.*, 1992; Wang *et al.*, 1994c; Gascoyne *et al.*, 1994b) and others (Holzel and Lamprecht, 1992) have contributed to the theory that allows dielectric data for particles to be derived from ROT.
The inventors have also analyzed the accuracy with which dielectric parameters can be derived from ROT analyses (Gascoyne *et al.*, 1994b). This allows us not only to understand essential dielectric and structural aspects of the microparticles but also, in conjunction with Eq. 4, to predict the microparticle electrokinetic behavior under all suspension conditions for all electrical field configurations. Analogous magnetic rotation experiments may also be conducted to characterize the microparticle magnetic properties.

**Dielectric Modeling of Engineered Microparticles**

The structure of an engineered microparticle such as that depicted in Fig. 1 and explained in accompanying text may be approximated, in terms of dielectric properties, by a spherical conductive interior (even if that interior, in turn, includes a dielectric core surrounded by a conductive shell) surrounded by a thin, poorly conducting shell. The

complex permittivity $\varepsilon_p^*$ of such a particle is given (Irimajari *et al.*, 1979; Huang *et al.*, 1992) by

$$\varepsilon_p^\bullet = \left\{ \frac{\left(\frac{r}{r-d}\right)^3 + 2\left(\frac{\varepsilon_{interior}^\bullet - \varepsilon_{shell}^\bullet}{\varepsilon_{interior}^\bullet + 2\varepsilon_{shell}^\bullet}\right)}{\left(\frac{r}{r-d}\right)^3 - \left(\frac{\varepsilon_{interior}^\bullet - \varepsilon_{shell}^\bullet}{\varepsilon_{interior}^\bullet + 2\varepsilon_{shell}^\bullet}\right)} \right\} \qquad (7)$$

where $\varepsilon_{interior}^*$ and $\varepsilon_{shell}^*$ are the complex permittivities conductivity and permitivities of the particle interior and the insulating shell, $r$ is the particle radius and $d$ is the thickness of the insulating layer. The cDEP and twDEP AC electrokinetic response of an engineered microparticle may be modeled in accordance with methodology known in the art using Eqs. 4 and 7.

FIG. 4 illustrates the cDEP and twDEP response of engineered microparticles with shell thickness varying between about 1 and 10 nm. It is evident from FIG. 4 that engineered microparticles of different compositions exhibit substantially different responses to AC electrical fields of various frequencies. The frequency response of a single microparticle type is referred to as a *dielectric fingerprint* and allows discrimination between microparticles having different structures.

Engineered microparticles with more complex dielectric fingerprints may be produced by applying multiple layers of materials of controlled thicknesses over a core material or by using a core material that is dispersive. The AC electrokinetic response of these more complex engineered microparticles may be predicted through the use of a multi-shell dielectric model known in the art, such as that described by Jones (1995), and incorporated herein by reference. In addition, other non-concentric structures may be produced and modeled by a spherical-shell equivalent.

A library of engineered microparticles with different dielectric fingerprints may be readily assembled by producing engineered microparticles with different physical compositions and structures. Microparticles having unique dielectric fingerprints may be individually addressable and may be used as frequency dependent handles to manipulate several different analytes in a sample mixture.

## Magnetophoresis

A particle of volume $\nu$ and magnetic permeability $\mu_p^*$ placed into an inhomogeneous magnetic field will experience a magnetophoretic force

$$F_{MAP} = 2\pi\mu_s R^3 k_{cm}(\mu_s^*, \mu_p^*, \omega_H) \nabla H(x,y,z)^2 \qquad (8)$$

where, $\mu_s$ is the magnetic permeability of the suspending medium, $R$ is the radius of the particle, $k_{cm}(\mu_s^*, \mu_p^*, \omega_H)$ is the Magnetic Clausius-Mossotti factor describing the magnetic polarizability of the particle with respect to its suspending medium, and $\nabla H(x,y,z)^2$ is the gradient of the square of the magnetic field strength. Here $\omega_H$ is the frequency of the applied magnetic field and will have the value zero for a static field. In analogy with the dielectric equation (Eq. 2), $\mu_s^*$ and $\mu_p^*$ are the complex permeabilities of the suspending medium and particle, respectively. In the case of a static magnetic field, these reduce to the real, static magnetic permeability parameters $\mu_s$ and $\mu_p$, respectively.

Note that equation 8 is the magnetic analog of equation 2. Alternatively, if the particle has a permanent volume magnetization $m$, then the magnetophoretic force will be

$$F_{MAP} = \mu_s R^3 m \nabla \cdot H(x,y,z)^2 \qquad (9)$$

It is possible for a particle to have both permanent and inducible magnetic polarization, components. In that case a combination of equations 8 and 9 may apply. For example, a particle may have a high permeability and at the same time demonstrate magnetic remnance. For a formal discussion of magnetophoresis, the reader is referred to Jones (1995).

The use of magnetophoresis to collect magnetically susceptible microparticles is well known in the art. Products from sources such Dynal, Inc. (Lake Success, NY) and Miltenyi Biotec (Auburn, CA) are routinely used for magnetophoresis-based separation techniques known as *immunomagnetic separation* (IMS) and *magnetically activated cell sorting* (MACS).

Although magnetic microparticles are readily available from many sources, the simultaneous exploitation of magnetic and dielectric microparticle properties for enhanced separations is a novel approach. The device used to discriminate, manipulate and/or isolate engineered microparticles contains both AC electrokinetic and magnetophoretic elements. For example, AC electrokinetic manipulation of engineered microparticles may include cDEP, twDEP, gDEP and ROT using an electrode array to which AC signals are switched. Magnetophoretic manipulation of engineered microparticles may be performed using a strong magnet fitted with a means for providing local magnetic field inhomogeneity in the vicinity of the electrode array. In such a device engineered microparticles experience both AC electrokinetic and magnetophoretic manipulating forces; both the dielectric and magnetic properties of the microparticles may thereby be exploited simultaneously to provide enhanced discrimination and manipulation capabilities.

**The Sensitivity of DEP-FFF to Changes in Particle Dielectric Properties**

The inventors have shown both theoretically and experimentally (Wang *et al*., 1998) that for a parallel electrode geometry, the dielectrophoretic levitation force for a dielectric particle suspended in a fluid medium falls off exponentially with height above the electrode. If the particle has a density such that it tends to sediment towards the electrode plane, a stable dielectrophoretic levitation will occur at an equilibrium height given by

$$h_{eq} = \frac{d}{2\pi} \ln \left\{ \frac{3\varepsilon_m U^2 Ap}{2(\rho_c - \rho_m)g} Re(f_{CM}) \right\}. \qquad (1)$$

Here $U$ is the electrical potential applied to the electrode array, A is a geometrical term, p is the proportion of the applied field that is unscreened by electrode polarization, $\varepsilon_m$ is the dielectric permittivity of the suspending medium, $(\rho_c - \rho_m)g$ is the sedimentation term, and dielectric polarization occurring at the particle-medium interface is described by the real part of the so-called Claussius-Mossotti factor $Re(f_{CM})$.

In DEP-FFF, the levitation occurs within a fluid that is flowing in a thin chamber according to a hydrodynamic flow profile. Thus the velocity of the particle will depend upon its levitation height $h_{eq}$ in the flow stream and particles having different values of $h_{eq}$ may consequently be separated from a starting mixture as they flow at differential velocities along the length of the chamber in which the levitation occurs.

In order to appreciate the dependency of a particle's velocity on small changes in its dielectric properties in a DEP-FFF experiment, it is helpful to write it first as a function of the equilibrium height prior to dielectric changes. Differentiating equation (1) with respect to the dielectric properties, we obtain

$$\partial h_{eq} = \frac{d}{4\pi} \cdot \frac{\partial Re(f_{CM})}{Re(f_{CM})}. \qquad (2)$$

If the hydrodynamic flow profile in the separation chamber is parabolic, then the particle velocity will depend on its levitation height according to

$$v_p = 6\langle v \rangle \frac{h_{eq}}{H} \left( 1 - \frac{h_{eq}}{H} \right) \qquad (3)$$

where H is the chamber thickness and $\langle v \rangle$ is the mean fluid velocity.

Thus we can write the incremental changes in velocity corresponding to incremental changes in height as

$$\partial v_p = 6\langle v \rangle \left[ \frac{1}{H} - \frac{2h_{eq}}{H^2} \right] \partial h_{eq}. \qquad (4)$$

We can further substitute from equation (3) for 6 (ν) to obtain

$$\partial h_{eq} = h_{eq} \left\{ \frac{H - h_{eq}}{H - 2h_{eq}} \right\} \cdot \frac{\partial v_p}{v_p} \qquad (5)$$

and then relate incremental changes in particle dielectric properties to corresponding changes in the particle velocity, using equation (2), as

$$\frac{\partial v_p}{v_p} = \frac{d}{4\pi h_{eq}} \cdot \left\{ \frac{H - 2h_{eq}}{H - h_{eq}} \right\} \cdot \frac{\partial \operatorname{Re}(f_{CM})}{\operatorname{Re}(f_{CM})} \qquad (6)$$

where, once again,

$$h_{eq} = \frac{d}{2\pi} \ln \left\{ \frac{3\varepsilon_m U^2 Ap}{2(\rho_c - \rho_m)g} \operatorname{Re}(f_{CM}) \right\}. \qquad (1)$$

Inspection of equation (6) reveals that the sensitivity of the particle velocity to incremental changes in height is largest when $h_{eq}$ and $\operatorname{Re}(f_{CM})$ are both small. The extreme sensitivity of the DEP-FFF phenomenon derives from the fact that these two conditions tend to be mutual. The inventors note, for example, from equation (1) that there is a threshold condition for levitation to occur at all,

$$\frac{3\varepsilon_m U^2 Ap}{2(\rho_c - \rho_{mc})g} \cdot \operatorname{Re}(f_{CM}) > 1$$

This asserts that the dielectrophoretic force must exceed the sedimentation force at the electrode plane for levitation. Clearly, for any prevailing conditions in which

$$\frac{A}{(\rho_c - \rho_{mc})g} \cdot \operatorname{Re}(f_{CM}) > 0,$$

the applied voltage U can be chosen in order to assure that levitation does indeed occur but that it is nevertheless small. We also notice that smaller magnitudes of $\operatorname{Re}(f_{CM})$ also assure smaller levitation heights. Of course there will be a practical limit to how large U can be and therefore how small $\operatorname{Re}(f_{CM})$ can be in a real application.

Because $h_{eq}$ and $\operatorname{Re}(f_{CM})$ dominate the denominator of equation (6), the sensitivity of the DEP-FFF incremental velocity to changes in the particle dielectric properties can therefore be very large if the appropriate conditions are chosen. In practice, changes in $\operatorname{Re}(f_{CM})$ as small as -0.0001 may be detectable - a truly astonishing sensitivity.

To illustrate this, we observe that in experiments conducted on human HL-60 leukemia cells on an electrode array having 20 micron electrode widths and spacings, the following parameters obtained:

$$A = -2.77 \times 10^{14} \text{ m}^{-3}, \, d = 80 \times 10^{-6} \text{ m}, \, p = 1, \, \rho_c = 1089 \text{ kg.m}^{-3},$$

$$\rho_m = 1033 \text{ kg.m}^{-3}, \, \varepsilon_m = 78 \, \varepsilon_m \text{ and } H = 200 \times 10^{-6} \text{ m}.$$

From equation (1), the levitation height is calculated as

$$h_{eq} = 6 \cdot 37 \times 10^{-6} \ln(-522 \operatorname{Re}(f_{CM})),$$

and the velocity sensitivity parameter is

$$\frac{\partial v_p}{v_p} = \frac{6 \cdot 37 \times 10^{-6}}{h_{eq}} \cdot \left\{ \frac{2 \times 10^{-4} - 2h_{eq}}{2 \times 10^{-4} - h_{eq}} \right\} \cdot \frac{\partial \operatorname{Re}(f_{CM})}{\operatorname{Re}(f_{CM})}.$$

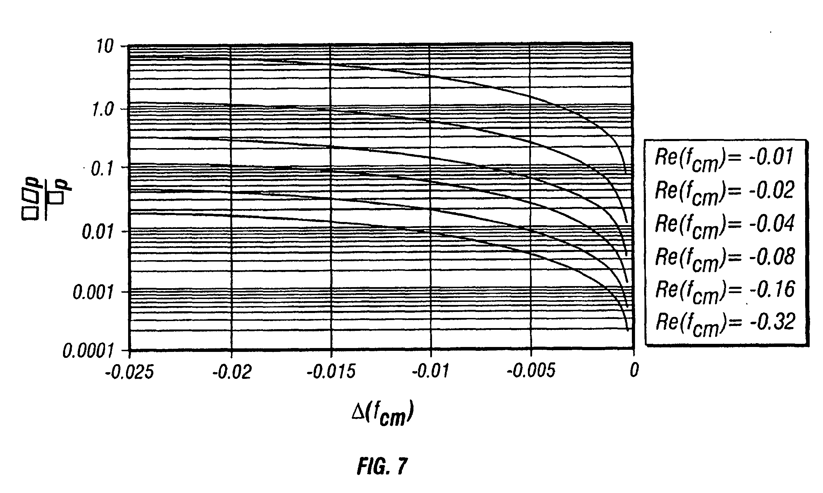

FIG. 7 shows, for these experimentally-verified parameters, the dependency of the particle velocity sensitivity, expressed as a % change in particle velocity, on small changes in $\operatorname{Re}(f_{CM})$ for starting values of -0·01, -0·02, -0·04, -0·08, -0·16 and -0·32. We note that an increment in the magnitude of $\operatorname{Re}(f_{CM})$ of 0.025, the maximum shown in the figure, is still considered to be extremely small.

FIG. 7 shows that if $\operatorname{Re}(f_{CM})$ of the particle/medium combination is small then the sensitivity of the DEP-FFF velocity to changes in dielectric properties is very large indeed. For example, for the starting value $\operatorname{Re}(f_{CM})$=-0·01, a change in $\operatorname{Re}(f_{CM})$ of -0.005 produces a 120% increase in the particle velocity (magenta curve). We can reliably measure changes in particle velocity as small as 2%, so the sensitivity is sufficient to detect a change in $\operatorname{Re}(f_{CM})$ of only -0.0001. On the other hand if the starting value of $\operatorname{Re}(f_{CM})$ is -0·32, detection of changes in the particle velocity will be reliable only for increments in $\operatorname{Re}(f_{CM})$ larger than about -0.03. For the highest level of sensitivity in a DEP-FFF detection assay, the dielectric particles and suspending medium should clearly be chosen so that $\operatorname{Re}(f_{CM}) \to 0$. We shall consider now, therefore, the conditions under which this can be achieved.

### The Claussius-Mossotti factor $f_{CM}$

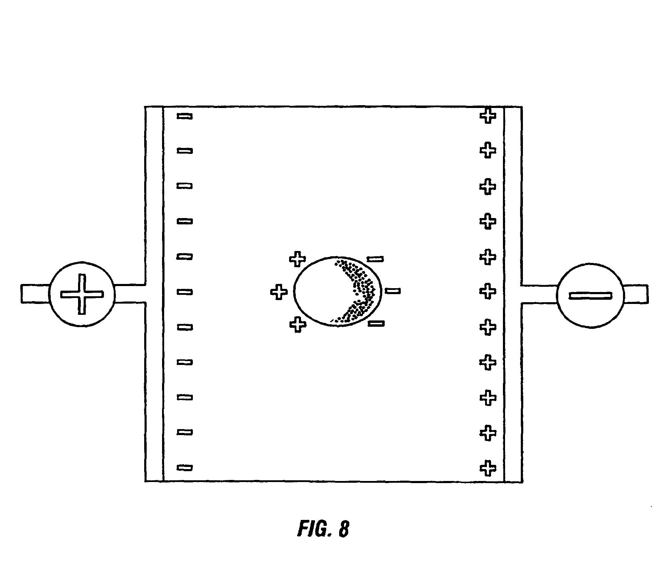

Consider a particle placed inside a dielectric medium to which an electric field has been applied. The dielectric suspending medium and particle will polarize in response to the field. However, if the dielectric properties of the particle are dissimilar from those of the suspending medium then the particle and medium will exhibit dissimilar degrees of polarization and the interface between the two will undergo a local polarization to ensure that the dielectric displacement D across the interface is continuous. In FIG. 8, the oval particle has a low dielectric permittivity and does not polarize appreciably. On the other hand the supporting medium, which has a high dielectric permittivity, polarizes and an interfacial polarization at the particle/medium interface arises to maintain continuity in dielectric displacement. This represents fairly accurately the case of an air bubble in aqueous suspension. It also represents a simple-minded view of a polymer bead suspended in water in a DEP-FFF experiment (but see later for very important discrepancies). The combination of all of the dielectric polarizations determines the dielectrophoretic response of the particle if the applied electrical field is inhomogeneous. The Claussius-Mossotti factor $f_{CM}$ expresses the *overall* polarizability of the particle in the suspending medium and $f_{CM}$ therefore includes polarization terms for both the particle and the medium. A central problem of AC electrokinetics is determining $f_{CM}$ for a given particle and medium combination so that the dielectrophoretic forces can be modeled.

For a spherical particle, the Claussius-Mossotti factor is given by

$$f_{CM} = \left( \frac{\varepsilon_p - \varepsilon_m}{\varepsilon_p + 2\varepsilon_m} \right)$$

where $\varepsilon_p$ is the effective permittivity of the particle and $\varepsilon_m$ is that of the medium. In the figure, the particle shown is non-polarizable so that $\varepsilon_p \ll \varepsilon_m$. In this case $f_{CM} \approx -0.5$ so that $\operatorname{Re}(f_{CM}) \approx -0.5$ also. In a dielectrophoretic experiment, this corresponds to the condition for maximum negative dielectrophoresis. Substituting this value for $\operatorname{Re}(f_{CM})$ into equation (1) and (6) gives shows that a huge change in the dielectric properties of the particle $\varepsilon_p$ would be required in order to create a substantial change in particle velocity under these conditions and one concludes that DEP-FFF would not be very sensitive for detecting alterations in particle dielectric properties. On the other hand we saw earlier that the DEP-FFF sensitivity increases as $\operatorname{Re}(f_{CM}) \to 0$. This implies that

$$\mathrm{Re}\!\left(\frac{\varepsilon_p - \varepsilon_m}{\varepsilon_p + 2\varepsilon_m}\right) \to 0$$

Optimizing DEP-FFF for detecting small responses in the dielectric properties of particles to target agents therefore boils down to exploring the conditions under which the particles and their suspending medium have very nearly the same effective relative permittivities.

**The Claussius-Mossotti factor for Engineered Microparticles**

Note that $\varepsilon_p$ is the *effective* particle permittivity. In the case of an air bubble, $\varepsilon_p$ could be modeled as a constant equal to the permittivity of free space. However, depending upon the structure and composition of the particle type in question, the expression for $\varepsilon_p$ and its corresponding dielectric model may be rather complex. For example, a simple model for an engineered microparticle such as that shown in Fig. 3, $\varepsilon_p$ as arises from a concentric shell system composed of a conductive interior having a permittivity $\varepsilon_c$, and a conductivity $\sigma_c$ surrounded by a very thin insulating layer having a permittivity $\varepsilon_s$ and a conductivity $\sigma_s$.

The corresponding effective permittivity of this so-called shell system is very frequency dependent. It is this frequency dependency that can be very effectively exploited to allows microparticles of different types to be characterized, discriminated and sorted by AC electrokinetic methods. Furthermore, appropriate suspending medium permittivity $\varepsilon_m$, conductivity $\sigma_m$, and applied frequency conditions can readily be selected so that the effective permittivity of an engineered microparticle type is very close to that of the suspending medium. In this way, microparticles lend themselves to high discrimination by DEP-FFF.

For engineered microparticles modeled after mammalian cells, the shell conductivity $\sigma_s$ will be extremely small and its influence in comparison to capacitance effects from $\varepsilon_s$ can be neglected. Conversely, for the frequency range, from 10 kHz to <1 MHz where capacitance effects are important, the influence of the conductivity of the suspending medium $\sigma_m$ is much greater than that of its permittivity $\varepsilon_m$. Under these conditions, the real part of the Claussius-Mossotti factor for engineered microparticles modeled after mammalian cells can be approximated as

$$\mathrm{Re}(f_{CM}) = \left\{ \frac{1}{(\sigma_m / 2\pi f)^2} - \frac{2}{(rC)^2} \right\}$$

Where $C$ is the specific membrane capacitance of the microparticle in F/m², $r$ is the radius of the microparticle and $f$ is the frequency of the applied field. We note that in this case the DEP crossover condition, where $Re(f_{CM}) \to 0$, occurs when

$$f = \frac{1}{\sqrt{2}\pi} \cdot \frac{\sigma_m}{rC}$$

The fundamental condition that has to be satisfied in order to allow for the discrimination of two different types of microparticles by DEP-FFF is that they be levitated to different heights. When the above approximation for the real part of the Claussius-Mossotti factor is valid, this condition can be expressed, using equation (1), as

$$\Delta H = \frac{d}{4\pi} \ln \left\{ \frac{\dfrac{1}{(\sigma_{m1}/2\pi f)^2} - \dfrac{2}{(r_1 C_1)^2}}{\dfrac{1}{(\sigma_{m2}/2\pi f)^2} - \dfrac{2}{(r_2 C_2)^2}} \cdot \frac{(\rho_{p2} - \rho_m)}{(\rho_{p1} - \rho_m)} \right\} \neq 0$$

where the subscripts 1 and 2 refer, respectively, to the two microparticles to be separated. It follows that

$$\frac{1}{(\sigma_{m_1}/2\pi f)^2} - \frac{2}{(r_1 C_1)^2}(\rho_{p2} - \rho_m) \neq \frac{1}{(\sigma_{m_2}/2\pi f)^2} - \frac{2}{(r_2 C_2)^2}(\rho_{p1} - \rho_m)$$

which can be satisfied in three ways, namely (1 and 2) if either of the two DEP crossover frequency terms defined within the square brackets approaches zero while the other is non-zero, a condition that can always be satisfied if

$$r_1 C_1 \neq r_2 C_2$$

or (3) when the frequency is far below either crossover frequency if

$$(\rho_{p1} - \rho_m) \neq (\rho_{p2} - \rho_m)$$

In this third case, microparticle size, density and shell capacitance all combine as factors to determine microparticle separability

## Examples

The following examples are included to demonstrate specific embodiments of the present disclosure. It should be appreciated by those of skill in the art that the techniques disclosed in the examples which follow represent techniques discovered by the inventor to function well in the practice of the invention, and thus can be considered to constitute specific modes for its practice.

### Example 1

### Engineered Microparticle Design Considerations

Life science research typically requires analysis of particles that range in size from about 100 nm to 10 μm in diameter. The main forces acting on particles in this size range are sedimentation forces and randomizing forces due to Brownian motion. For a particle of radius 1 μm and density of 1.05 g/cm$^3$ suspended in aqueous medium ($\rho$ = 1.00 g/cm$^3$) at 25 °C the sedimentation and Brownian forces each have magnitude of approximately 2 x 10$^{-15}$ N.

To effectively use conventional dielectrophoresis as a manipulating force, the cDEP force must be greater than the other forces acting on the particle, and in one embodiment, about an order of magnitude greater than the other forces acting on the particle. According to Eq. 4, if Re($f_{CM}$) = 0.5, then $\nabla E(rms)^2$ should be approximately 9 x 10$^{12}$ V$^2$/m$^3$ to give a cDEP force that is ten times greater than the sedimentation or Brownian forces (Pething and Markx, 1997).

Using microelectrodes, and methodology known in the art, it is possible to generate fields of this magnitude with an applied voltage of about 10 V or less. The sedimentation and conventional dielectrophoretic forces are both proportional to r$^3$, while the randomizing forces of Brownian motion are proportional to r$^1$. For particles larger than 1 μm, the sedimentation and cDEP forces are the dominant forces acting on particles, and conventional dielectrophoresis maybe used to manipulate particles about 10 μm in diameter or larger. For smaller particles, Brownian motion forces dominate over sedimentation forces. By using electrodes with submicron geometries, one may generate cDEP forces capable of manipulating viruses and other particles that are about 100 nm in diameter or smaller (Muller *et al.*, 1996).

With the benefit of this disclosure, engineered microparticles may be designed with properties that make them amenable to AC electrokinetic and magnetophoretic manipulation. As discussed previously for the specific case of cDEP manipulation of particles, the magnitude of the cDEP force must be sufficient to overcome the influence of the other forces acting on the particles in the system. This is true for any AC electrokinetic or magnetophoretic manipulation. The forces acting on particles are generally sedimentation forces and Brownian motion induced randomizing forces. Since the magnitude of these forces depends upon the particle properties, the engineered microparticles may be designed so that the effect of the AC electrokinetic force is maximized by appropriately scaling the influence of competing forces.

Sedimentation forces may be scaled, for example, by producing engineered microparticles with an effective density between about 1.0 and 2.0 $g/cm^3$. A small ($< 1.5 \times 10^{-12}$ N) sedimentation force will then act on 10 $\mu$m particles of this density when they are suspended in an aqueous medium. For most applications this characteristic is preferred, as microfabricated AC electrokinetic devices are typically designed with microelectrodes on the lower surface of the device. The electric field strength, and therefore the AC electrokinetic force, is most pronounced near the electrode plane.

Negative buoyancy may be used to ensure that the microparticles fall to the electrode plane where they can be held by positive cDEP or levitated by negative cDEP. The Brownian forces may be reduced by designing microparticles that are about 10-20 $\mu$m in diameter. The influence of Brownian forces on particles of this size is negligible when compared to the magnitude of the DEP forces that are typically used for the manipulation of engineered microparticles. Analogous arguments are applicable to the design of the magnetic microparticle properties and the magnetic field.

One approach to achieve a final effective density in a specified range may be as follows: custom engineered microparticles may be produced by thin-film deposition of conductive, insulating and/or magnetically susceptible materials on low density ($< 1.3$ $g/cm^3$) spherical substrates. It is known from Maxwell's laws of electromagnetism that a conductive spherical shell is indistinguishable from a solid conducting sphere in response to an externally applied electrical field. The conductive layer may be a thin-film of metal such as gold, silver, platinum or copper about 10-100 nm thick.

This layer may be applied over the substrate by physical vapor deposition (PVD) or electroless plating (Elshabini and Barlow, 1998) according to principles known in the art, to form the conductive core. The insulating material may be a thin film of metal oxide such as $Al_2O_3$ or polymer material such as polystyrene or PTFE. Such materials may be applied through PVD or microencapsulation (Lim, 1984) techniques, for example.

Since the densities of the conductive and insulating layers may range from 8.9-21.4 $g/cm^3$ and 1.1-2.2 $g/cm^3$ the corresponding substrate must have a low density to yield a finished microparticle in the desired density range. Polystyrene and hollow glass microparticles about 10-1000 $\mu$m in diameter are commercially available with a density of approximately 1.0 $g/cm^3$ from several companies including Dynal, Inc. (Lake Success, NY), Miltenyi Biotec (Auburn, CA), Cortex Biochem, Inc. (San Leandro, CA), and BioSource International (Camarillo, CA).

Similar considerations apply to the design of microparticle magnetic properties such that appropriate microparticle density may be achieved. Suitable magnetic materials for microparticle core or layer construction include ferrites, rare-earth containing ceramics and glasses, as well as iron, cobalt, titanium and other materials containing atoms or molecules with uncompensated electron spins.

Characteristics that determine the dielectric properties of microparticles include their size, surface charge, density, composition and electrical conductivity. With the benefit of this disclosure and technology known in the art, all of these parameters may be modified in order to achieve a desired dielectric fingerprint. In particular, engineered microparticles may be fabricated so as to incorporate defined surface and internal dielectric, magnetic, and density properties through the use coatings, internal and surface layers and internal compartments and/or cores, each of which may be dielectric, conductive, magnetic or non-magnetic. The dielectric and electrical properties of the microparticle surface and of each coating, layer, compartment and core may be different. The overall dielectric and magnetic properties of a microparticle will be determined by the synergistic dielectric and electrical contributions of each of its component parts and by the presence in them of magnetically susceptible materials. By combining structural elements having appropriate dielectric and electrical properties, different types of microparticles may be synthesized that have distinct and distinguishable dielectric properties.

The physics of dielectrics and magnetics makes it possible to manufacture, in more than one way, microparticles having essentially similar dielectric and/or magnetic properties. For the purpose of this invention, all microparticles having similar dielectric and magnetic properties in a defined frequency range of interest shall be considered as being identical microparticles even if their underlying physical compositions are different.

Examples of microparticle structures include simple spheres of latex, metal, glass, semiconductor, plastic or magnetic materials, with or without controlled surface properties or coatings. More complex structures include microparticles having one or more of the following features: (i) an electrically non-conductive membrane-like coating with an electrically more conductive interior; (ii) a layer or core containing a dielectric material having a dielectric dispersion within a frequency range of interest; (iii) a highly conductive surface layer, or core; (iv) a surface with a net charge that contributes to the properties of the microparticle through interaction with a dielectric medium; (v) one or more layers or a core possessing magnetic susceptibility. The present disclosure concerns any microparticle type whose overall dielectric and magnetic properties are specifically chosen such that the microparticles may be used for isolation, identification, characterization, or other manipulation of target analytes through AC electrokinetic or combined AC electrokinetic and magnetic methods.

**Example 2**

**Experimental Studies**

Silver-coated, hollow glass spheres were obtained from Potters Industries (Valley Forge, PA) and custom encapsulated in varying thicknesses of polystyrene by Theis Technology (St. Louis, MO) using a surfactant-free microencapsulation protocol. The resulting microparticle structure was similar to that depicted in FIG. 1. Upon application of an inhomogeneous electric field from a castellated, interdigitated electrode array, microparticle manipulation was accomplished by switching the field frequency and voltage. Dielectric responses varied in accordance with the predictions of Eqs. 4 and 7. The results confirm the analysis presented here and indicate that both the dielectric and conductive properties of the polystyrene coating define microparticle behavior as expected. Experiments using dielectric ferrite microparticles from Dynal, Inc. (Lake Success, NY) also confirmed that magnetic and DEP forces may be used simultaneously for microparticle manipulations.

**Example 3**

**Applications of Engineered Microparticle Technology**

The utility of microparticle-based technologies for the identification, manipulation and isolation of target cells is universal. Recently, the use of microparticles in molecular biology has become widespread and promises to redefine the methodologies employed in life sciences studies wherever cell or molecular targeting or recognition is required. Yet current approaches are one-dimensional and offer little flexibility. For instance, parallel probing of multiple targets is not possible, targets may only be attracted to a collection site so that negative selection (the preference in some sorting applications) is difficult if not impossible, and sorting is essentially digital (targets cannot be discriminated according to binding efficiencies but only according to whether or not they bind any number of microparticles ranging from one to tens of thousands). The methods described here overcome these limitations and offer the potential for separating several targets simultaneously from a mixture, for using both positive and negative selection to greatly enhance the purity of isolated fractions, and for allowing targets to be discriminated according to their binding efficiencies (thus, cells could be sorted according to the *number* of antibody binding sites on their surfaces rather than just according to whether or not they had *any* binding sites).

Immediate applications for the engineered microparticle technology include:

1) sorting cells according to the concentration of surface markers including the CD antigens, growth factor receptors, and/or other membrane-associated proteins or moieties;
2) isolation of blood cell subpopulations of high purity;
3) removal of tumor cells and/or T-cells from stem cell harvests;
4) isolation and identification of pathogens from blood, urine and other patient samples;
5) isolation and identification of pathogens in public water supplies and in food preparation and processing facilities;
6) isolation of subcellular compartments such as vesicles and organelles;
7) isolation and identification of nucleic acids, proteins, and other biomolecules from biological samples including those of extremely small volume.

**Example 4**

**Engineered Microparticle Technology for Analyte Separation and Identification Based on *Cluster of Differentiation*, or *CD,* Antigens Found on the Surface of Cells**

Three different microparticle types are engineered such that they each have different cDEP behavior as labeled *a, b* and *c* in Fig. 4. By linking a probe for CD3 to engineered microparticles with cDEP behavior labeled *a*, a probe for CD4 to microparticles with cDEP behavior labeled *b*, and a probe for CD18 to microparticles with cDEP behavior labeled *c*, three different engineered microparticle labels may be made. A mixture of these three different labels may then be used to simultaneously label a blood sample containing many different cell subpopulations in a *single* labeling step.

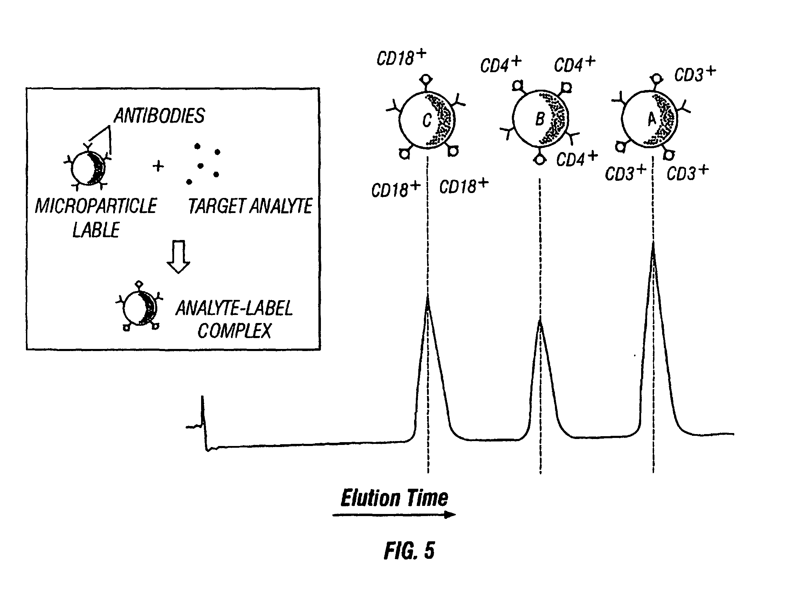

Using an AC electrokinetic-based separation method such as DEP-FFF, the three different microparticle types, and thus $CD3^+$, $CD4^+$ and $CD18^+$ cells, may be separated in a single DEP-FFF separation step (FIG. 5). Using this method, analysis of cell subpopulations not distinguishable from other subpopulations by their size, density or surface specific capacitance characteristics alone is made accessible.

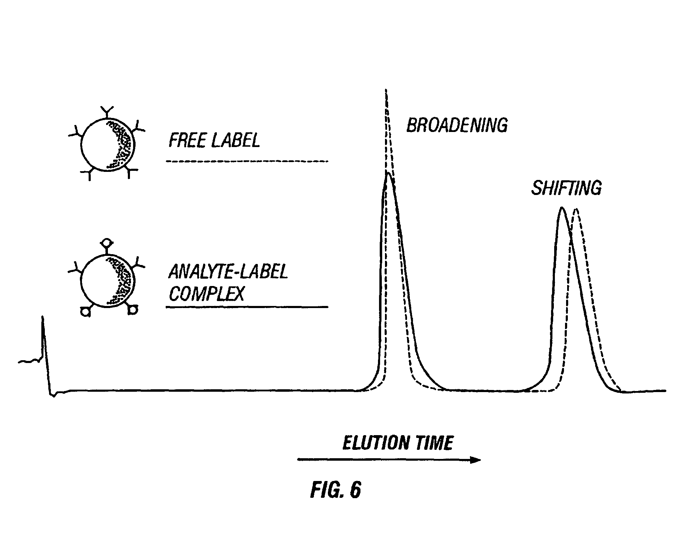

In a DEP-FFF separation, the different microparticle types fractionate into well-defined bands, each of which emerges as a single, well-defined elution peak (FIG. 5). In the case of free, unbound microparticle labels, the peak shape is relatively narrow and sharp. However, because the dielectric properties of the microparticles are perturbed upon analyte binding, the peak shape of analyte-label complexes is broader and/or exhibits a shift in elution time (FIG. 6). The extent of this perturbation may be dependent upon the nature of the analyte and the extent of analyte binding. It should be noted that such elution peak changes may be used as the basis for quantitative methods of analyte detection.

**Example 5**

**Engineered microparticles**

Microparticles may be fabricated using self-assembled monolayers (SAMs) of alkanethiolate on silver or gold metalized hollow glass cores. Alkanethiols $CH_3(CH_2)_nSH$ chemisorb spontaneously onto gold surfaces to form alkanethiolates: $X(CH_2)_nSH + Au^0 \zeta X(CH_2)_nS^-Au^I + \frac{1}{2}H_2$. The alkanethiolates self-organize into densely packed, robust monolayer film. Such films have been extensively characterized, and their insulating properties established. The thickness of an alkanethiol SAM film is dependent upon the number n of methylene groups in the alkyl chain. The dielectric properties of engineered microparticles made with different alkane chain lengths may be investigated. Experiments may be performed with hybrid bilayer membranes formed by the fusion of lipid vesicles with self-assembled alkanethiolate monolayers. The effects of altering the alkanethiol head group *X* may be determined. In addition other molecules may be adsorbed, such as thiolated DNA to the metalized microparticle surface. Finally, protocols may be developed for linking protein and oligonucleotide capture probes the alkanethiolate head groups.

**Example 6**

**Detection of Chemical Biological Warfare Agents**

The engineered microparticles discussed herein may be applicable to an immense range of assays extending from detection of CBW agents to detection of medical, chemical, agricultural and environmental analytes. A microparticle-based sandwich assay may be developed to detect specific protein simulants such as cholera toxin β-subunit (CTB) and staphylococcal enterotoxin B (SEB). In addition a microparticle-based sandwich assay may be developed for tumor necrosis factor (TNF), an early indicator of a challenged host immune system. Monoclonal antibodies directed against these proteins are available and may be used to construct the capture and labeling probes. Engineered microparticle-based sandwich assays may be developed to detect specific nucleic acid sequences derived from bacterial simulants such as *Bacillus subtilis* and *Escherichia coli* serotype O157:H7. Oligonucleotide probes that are complimentary to mRNA sequences found in these organisms are readily obtained. By utilizing dielectrophoresis to focus the analyte-microparticle complexes into a densely packed spherical region, the local analyte concentration may be raised by several orders of magnitude, eliminating the need for nucleic acid amplification, and increasing the assay sensitivity. Existing one-pot assays may be adapted to the PFP platform. Good candidates for adaptation include the bicinchoninic acid (BCA) protein assay from Pierce and the LIVE/DEAD viability/cytotoxicity assay from Molecular Probes.

**Example 7**

**Indexing**

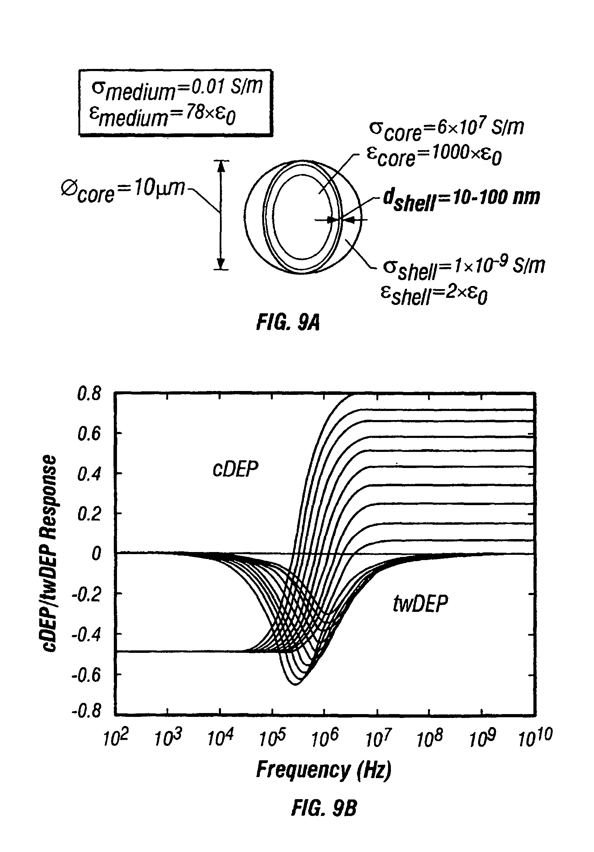

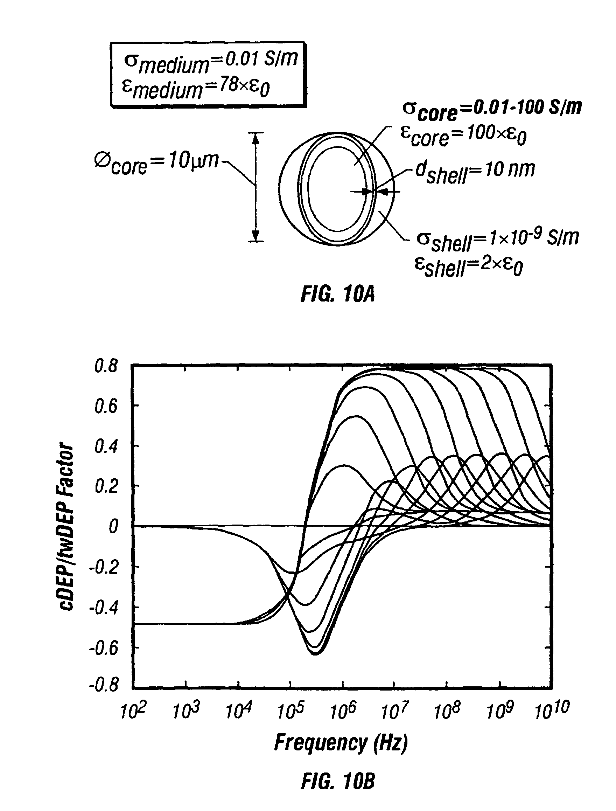

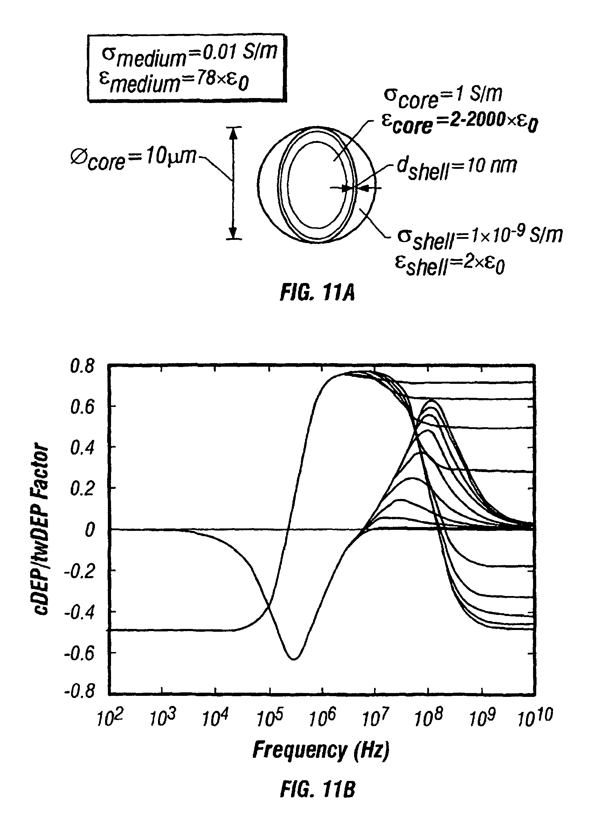

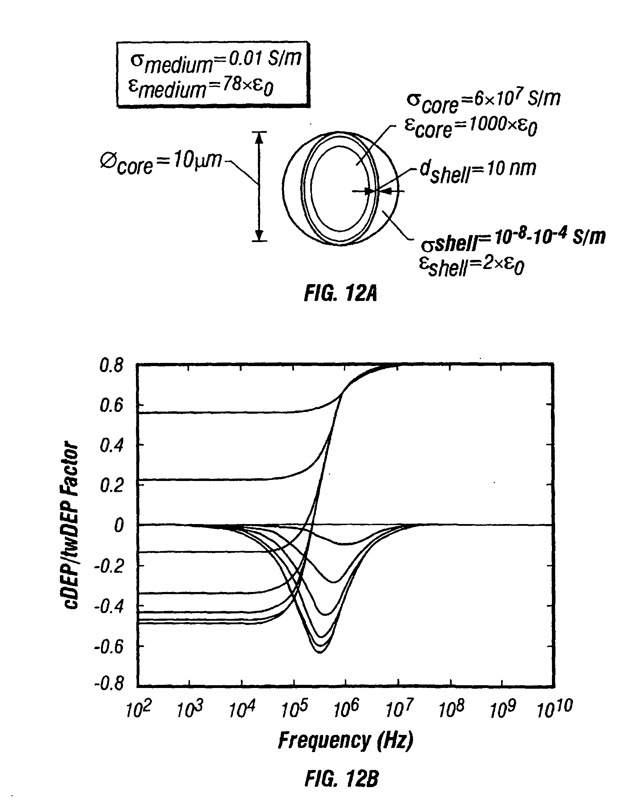

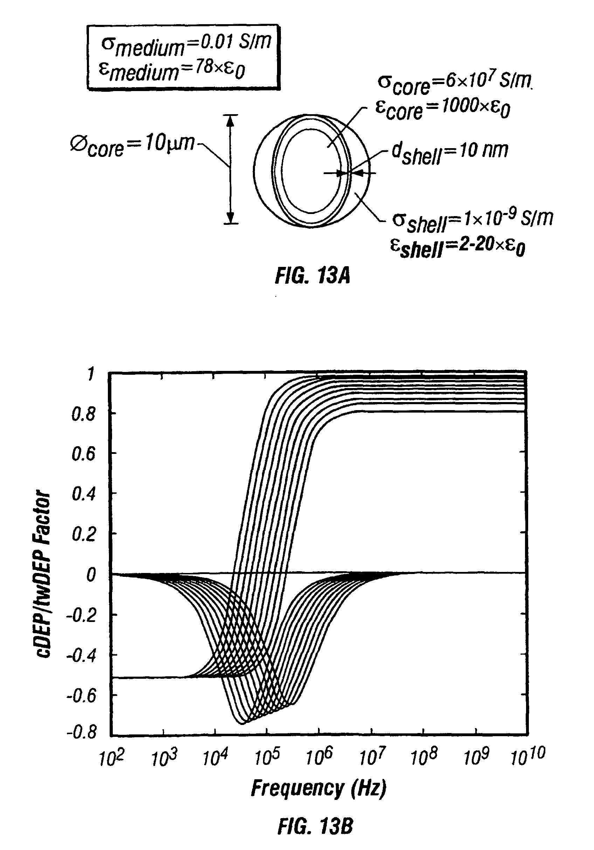

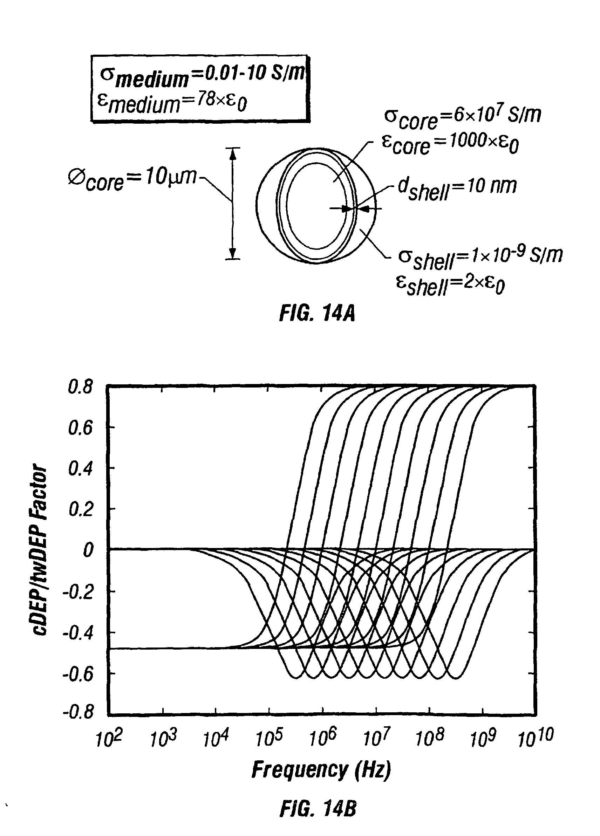

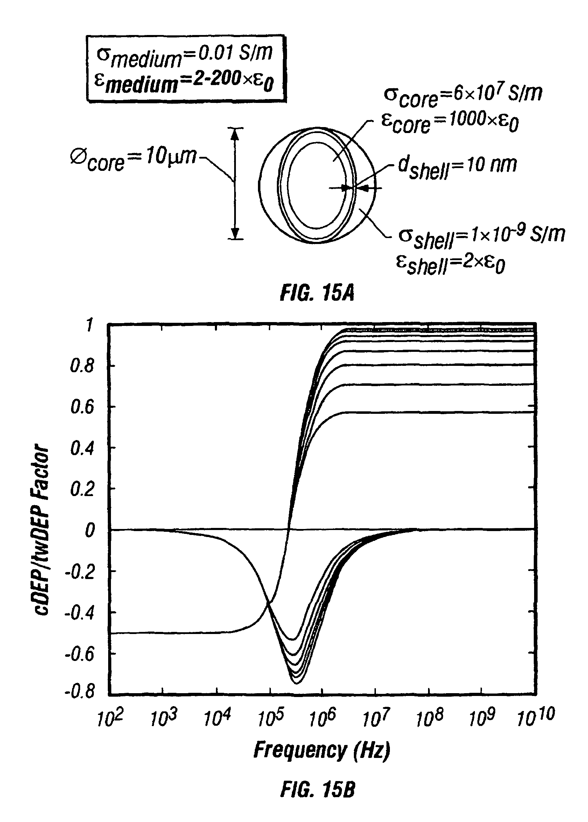

FIGS. 9-15 show engineered microparticles having different dielectric properties. Shown also are the response versus frequency relationship for these particles.

In one embodiment, these particles may be used as an indexing library. In particular, each different microparticle may be made to bind to a different analyte and then simultaneously manipulated, identified, sensed, and detected according to the different response characteristics of the library shown in FIGS. 9B, 10B, 11B, 12B, 13B, 14B, & 15B.

**Example 8**

**Sandwich Assays**

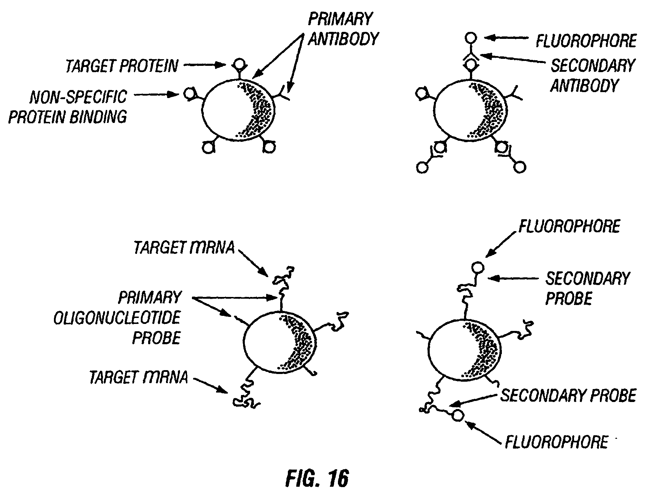

FIG. 16 is a schematic illustrating sandwich (double label) assays that may be used for detecting protein and mRNA in studies in accordance with the present disclosure. As illustrated, the engineered microparticles of FIG. 16 may include linking elements designed to interact with proteins and/or mRNA. Labels, such as fluorophores or bioluminescence labels, may act as secondary probes.

With the benefit of the present disclosure, those having skill in the art will appreciate that the engineered microparticles of FIG. 16, along with the target analytes (and labels) attached thereto may be manipulated using dielectrophoretic forces. In particular, the complexes of FIG. 16 may be sorted, separated, trapped, sorted and generally processed using dielectrophoresis. This processing may take place on a reaction surface such as the surface disclosed in pending United States Application No. 09/249,955, filed February 12, 1999, and entitled, "Method And Apparatus for Programmable Fluidic Processing," which has already been incorporated herein by reference and/or the field-flow fractionation device disclosed in United States Patent No. 5,993,630 which has also been incorporated by reference. A specific example of processing that may be done on a reaction surface is using dielectrophoresis to (a) pull complexes such as those shown in FIG. 16 from a solution, and (b) process those complexes upon the reaction surface.

The formation of the complexes of FIG. 16 may be detected by noting the difference in dielectric properties before and after the formation of the complex. This difference may be measured using one or impedance sensors known in the art or any other methodology known in the art for measuring dielectric, electrical, or physical properties. Plasmon resonance is an example of one such methodology.

According to one embodiment of the present disclosure, different engineered microparticles may be manufactured so that each microparticle has different dielectric properties. For instance, each engineered microparticle may be made with a different thickness and/or composition of its insulating layer(s). Together, this group of microparticles may form a library.

To each different microparticle of the library, a different linking element may be applied. The microparticles may then be admixed with a sample containing one or more target analytes to form one or more complexes. The dielectric properties of each microparticle may be distinguished from the dielectric properties of its corresponding complex using suitable impedance sensors. This distinguishing of dielectric properties allows one to detect if a complex has been formed. Further, the dielectric properties of each microparticle may be distinguished from one another. This distinguishing allows one to determine the identity of the microparticle being detected.

**Example 9**

**Engineered Microparticles with one or more self-assembled monolayers**

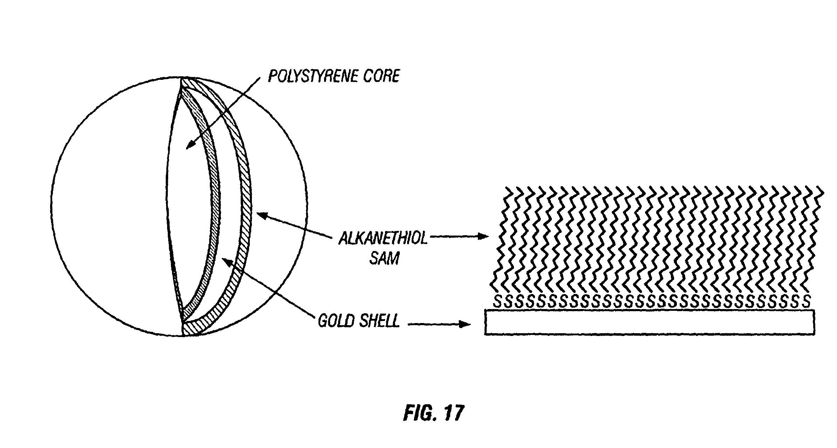

FIGS. 17-21 show multi-layered engineered microparticles according to embodiments of the present disclosure.

FIG. 17 shows a single layer engineered microparticle. It includes a polystyrene core coated with a conductive gold shell. An insulator such as a self-assembled monolayer (an alkanethiol self-assembled monolayer is illustrated) may coat the conductive gold shell. By varying, for example, the size and/or composition of the insulating layer and/or the conductive layer, one may vary the dielectric properties of the engineered microparticle.

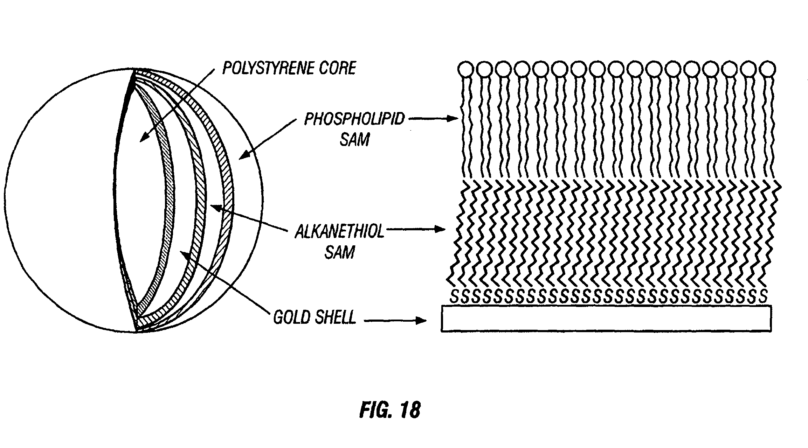

In FIG. 18, a two-layered engineered microparticle is shown. It comprises the following layers: a polystyrene core, a gold shell, an alkanethiol self-assembled monolayer, and a phospholipid self-assembled monolayer.

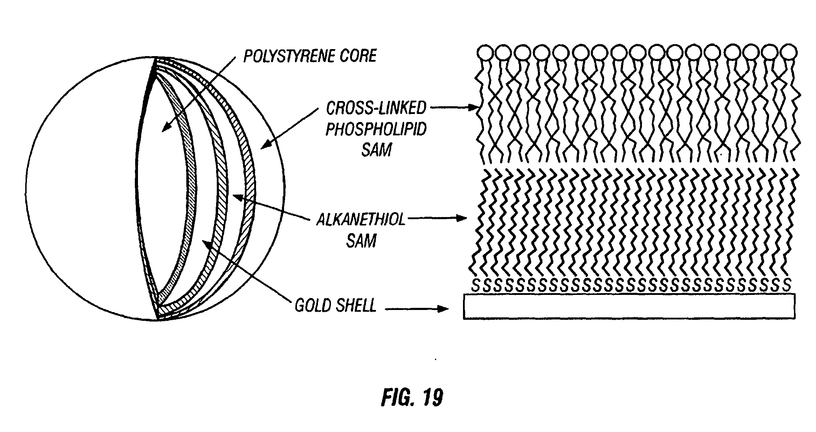

In FIG. 19, the phospholipid self-assembled monolayer is cross-linked. When oxidized, the unsaturated lipids cross-link to form polymer structures. Since it is cross-linked, the phospholipid layer, which has a hydrophilic "head" and hydrophobic "tail," is more stable in organic solvents.

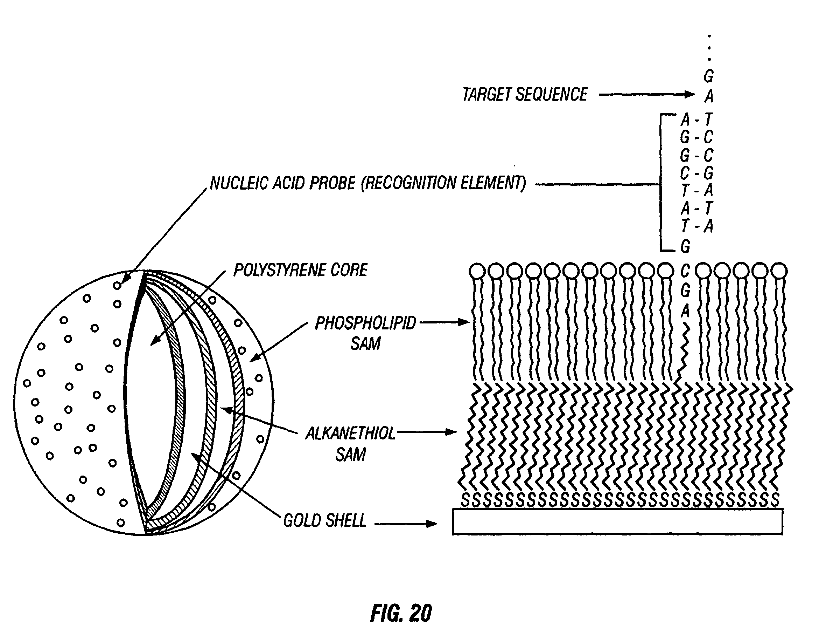

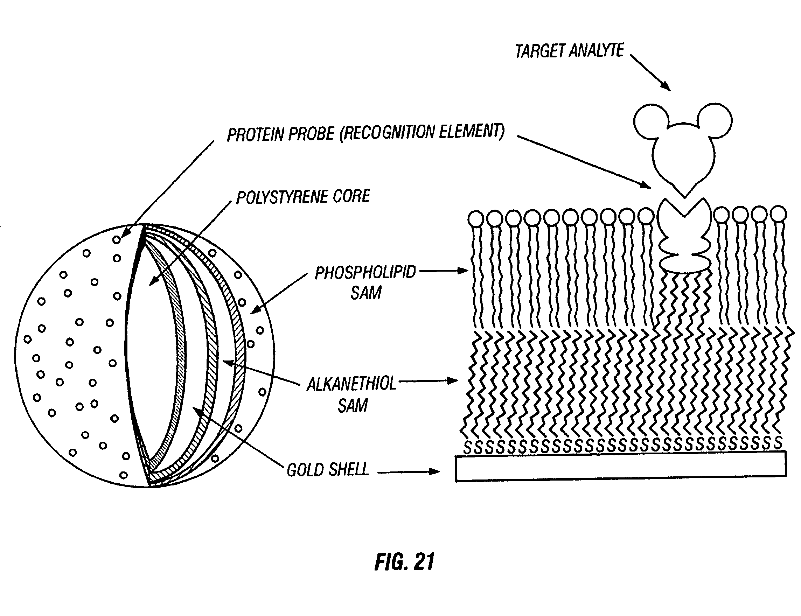

FIGS. 20 and 21 show two-layered engineered microparticles including linking elements. In FIG. 20, the linking element is a nucleic acid probe. In FIG. 21, it is a protein probe. In each of these figures, the linking element is bound to the gold conductive core. However, it will be understood that the linking elements may be bound to the outer or inner insulating layer.

With the benefit of this disclosure, it will be apparent that the microparticles of FIGS. 17-21 may include more or fewer layers. For instance, beyond the gold shell layer (which need not be solid) and the SAM layer(s) shown, one or more additional SAMs or other layers may be added. One or more of the layers may be crosslinked, and one or more labels may be added to the linking elements.

Further, one will understand that the conductive gold shell of FIGS. 17-21 may be substituted with any suitable conductor, including conductive polymers or the like. Additionally, the polystyrene core may be substituted with any other suitable material.

**Example 10**

**Fabrication Considerations**

The alkanethiolates disclosed herein self-organize reliably into robust, densely packed monolayer films of reproducible thickness. Additionally, biomimetic hybrid bilayer membranes (HBM's) can be formed by fusing phospholipid vesicles with engineered microparticle cores that have already been coated with alkanethiolate monolayers. The thickness of the insulating layer surrounding the core of an engineered dielectric microparticle is dependent on the both the number of methylene groups in the alkyl chain of the alkanethiol SAM film and the number of methylene groups in the lipid tail of the phospholipid used to form the hybrid bilayer membrane.

Therefore, one may produce a library of particles with insulating layers of different thicknesses and different dielectric properties by simply changing the length of the hydrocarbon chain in the alkanethiolate and phospholipid layers of an the engineered microparticles shown herein.

Gold-coated polystyrene microparticles may be obtained from Dynal Biotech that are uniform (coefficient of variation < 5 %) 9.6 μm in diameter with a density of 2.2 g/cm$^3$. The inventors have constructed four different types of engineered dielectric microparticles by forming self-assembling monolayers of alkanethiolate and phospholipid on the gold-coated polystyrene core particles. Engineered microparticles with a relatively thin insulating layer have been made coating the core particles with a single alkanethiolate monolayer of:

(i) nonyl mercaptan [$CH_3(CH_2)_8$-SH] to give a **$C_9$** insulating layer,
or
(ii) octadecyl mercaptan [$CH_3(CH_2)_{17}$-SH] to give a **$C_{18}$** insulating layer.

Engineered microparticles with thicker insulating layers have been made by forming a second insulating monolayer of DMPC [1,2-Dimyristoyl-*sn*-Glycero-3-Phospbocholine ] phospholipid over the alkanethiolate layer as follows:

(iii) nonyl mercaptan *plus* DMPC to give a **$C_{23}$** insulating layer,
or
(iv) octadecyl mercaptan *plus* DMPC to give a **$C_{32}$** insulating layer.

Each sample of engineered dielectric microparticles was made by first washing 10 mg of the gold-coated core microparticles in 10 ml of absolute ethanol in a glass tube to clean the gold surface of microparticles. After several minutes of mixing, the microparticles were pelleted by centrifugation using a bench-top centrifuge, the ethanol was decanted off and the washed microparticles were and combined with 10 ml of a 1 mM solution of nonyl or octadecyl mercaptan in absolute ethanol. This suspension was gently mixed for at least 12 hours to ensure the formation of a well-organized, high-integrity self-assembled monolayer (SAM).

The adsorption process for moderate concentrations (1 mM) of alkanethiol is characterized by a rapid initial phase during which the alkanethiol thickness rises to 80-90% of its maximum within a few minutes. This initial phase is followed by a slower period, lasting several hours, during which the alkanethiolate layer achieves its final thickness. It has been reported in the art that monolayers of alkanethiols on gold appear to be stable indefinitely in air or in contact with liquid water or ethanol at room temperature. The alkanethiolate-coated microparticles were recovered by centrifugation, washed twice in absolute ethanol and twice in triple-distilled water and stored at 4 °C in 1 ml of triple-distilled water.

The phospholipid layer was formed over the alkanethiol layer by combining alkanethiolate-coated microparticles with aqueous suspensions of DMPC small unilamellar vesicles. The DMPC vesicles were made by placing 1 ml of a 20 mg /ml DMPC in chloroform lipid solution into a round bottom flask and evaporating the solvent for several hours using a vacuum rotary evaporator. The dried lipid was resuspended in 50 μl of isopropanol and injected into 10 ml triple-distilled water while vortexing to form a suspension of large multilamellar vesicles. The solution of large vesicles was sonicated in a bath sonicator for several minutes to disrupt the large vesicles into small 20-100 nm unilamellar vesicles. This 10 ml suspension of small vesicles was combined with 10 mg of alkanethiolate-coated beads and gently mixed for 30 minutes at room temperature. The alkanethiolate-phospholipid-coated microparticles were recovered by centrifugation, washed twice in triple-distilled water and stored in triple-distilled water.

**Example 11**

**Engineered Microparticle Testing**

The analysis of the single-shell dielectric model predicts a definite relationship between the thickness of the outer insulating shell and the dielectrophoretic properties of an engineered dielectric microparticle. Engineered microparticles of appropriate thin-insulating-shell-over-conductive-interior composition are predicted to experience strong negative dielectrophoresis at frequencies between $10^2$-$10^4$ Hz. In this frequency range, the electrical field would be unable to penetrate the outer insulating shell — from a dielectric perspective, the microparticle would have a high AC impedance and appear relatively non-polarizable. At frequencies in the $10^7$-$10^9$ Hz range, engineered microparticles are predicted to experience strong positive dielectrophoresis. In this frequency range, the electrical field would penetrate the thin outer insulating shell via capacitive coupling and the core properties would dominate — dielectrically, the microparticle would have a low AC impedance and appear highly polarizable.

In the transitional $10^5$-$10^6$ Hz range, increasing frequency is predicted to correlate with a change in the dielectrophoretic force acting on the engineered microparticle from decreasing negative to increasing positive. At the *crossover frequency, $f_c$,* the net dielectrophoretic force acting on the microparticle is zero — at frequencies below $f_c$, the particle experiences negative dielectrophoresis, and at frequencies above $f_c$, the particle experiences positive dielectrophoresis. An increase in the thickness of the insulating outer shell correlates with an increase in the crossover frequency. The relationship between the thickness of the insulating shell and the crossover frequency is given by the following relationships

$$C_{mem} \approx \frac{\sigma_s}{R\pi\sqrt{2}f_c}$$

and

$$C_{mem} = \frac{\varepsilon_0 \varepsilon_{mem}}{d}$$

where $C_{mem}$ is the specific membrane capacitance (i.e., normalized to the shell area), $\sigma_s$ is the conductivity of the suspending medium, $R$ is the radius of the engineered microparticle, $f_c$ is the crossover frequency, $\varepsilon_0$ is the permittivity of free space, $\varepsilon_{mem}$ is the permittivity of the insulating layer, and $d$ is the thickness of the insulating layer. Combining these equations yields the following relationship:

$$d \propto \frac{f_c}{\sigma_s}$$

According to the equation above, the inverse slope of a plot of $f_c$ versus $\sigma_s$ gives the approximate specific membrane capacitance for a given engineered microparticle type. Furthermore, the slope of such a plot should increase with increasing membrane thickness.

Dielectrophoretic crossover frequency studies were performed by the inventors in order to determine whether four types of engineered dielectric microparticles (outer self assembled insulating monolayer of $C_9$ or $C_{18}$ alkanethiol or $C_9$ or $C_{18}$ alkanethiol + $C_{14}$ phospholipid) exhibited the predicted correlation between insulating shell thickness and crossover frequency. For the dielectrophoretic studies, the engineered dielectric microparticles were suspended in a DEP buffer containing 8.5 % (w/v) sucrose, 0.3% (w/v) dextrose. The electrical conductivity of the buffer was adjusted with 300 mM EDTA (adjusted to pH 7.0 with NaOH). Aliquots of the engineered bead suspension were placed in an open reservoir above parallel electrode (50 µm trace - 50 µm gap) of gold-on-glass construction that was energized with 1 - 10 volts peak-to-peak at frequencies between 1 kHz and 100 kHz to generate inhomogeneous electric fields for dielectrophoretic manipulation. Dielectrophoretic manipulation was accomplished by switching the field frequency, and the dielectrophoretic crossover frequency was determined.

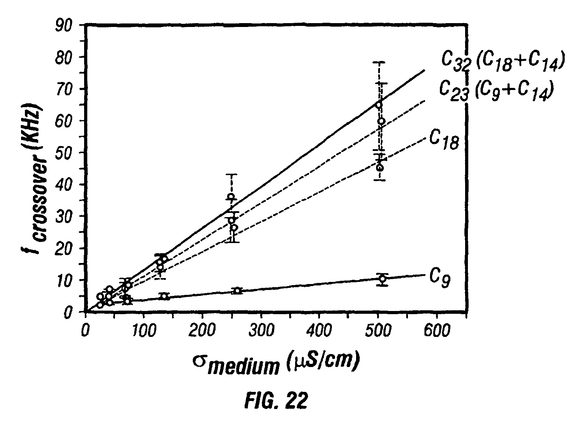

As shown by FIG. 22, the engineered microparticles show a definite dependence on thickness of the insulating outer shell as mediated by the choice of an alkanethiol and phospholipid of appropriate carbon chain length. Furthermore, the y-intercept occurs at very near zero, indicating the conductivity of the insulating layer is low and the self assembled monolayers provide a robust, uniform insulating layer.

Lipophilic molecules such as the pore-forming protein mellitin may be incorporated into the insulating layer. In addition, one may adsorb probes such as thiolated nucleic acids and proteins to the gold surface of the engineered microparticle core, and oligonucleotide and protein capture probes may be linked to the alkanethiolate and phospholipid molecules.

## <u>REFERENCES</u>

Abidor, Li, Sowers, "Membrane electrofusion yeilds in membrane fractions obtained with a colloid-osmotic hemolysis and electrohemolysis procedure," *Bioelectrochem. Bioenerget.* 34, 31-37, 1994.

Arnold, Zimmermann, "Electro-rotation: developments of a technique for dielectric measurements on individual cells and particles," *J. Electrost.* 21 151-191, 1988.

Arnold, Zimmermann, "Rotation of an isolated cell in a rotating electric field," *Naturwissenschaften* 69 297-300, 1982.

Barany, *Proc. Natl. Acad Sci. USA* 88:189, 1991.

Dutton *et al., Proc. Natl. Acad. Sci.,* 76, 3392-3396, 1979.

Elshabini, Barlow, in Thin Film Technology Handbook, McGraw-Hill, New York, Ch 1, 1998.

Fuhr, "Über die rotation dielektrischer körper in rotierenden feldern," PhD Dissertation Humboldt-Universität, Berlin Chap. 3, pp24-53, 1985.

Fuhr, Rosch, Müller, Dressler, Goring, "Dielectric spectroscopy of chloroplasts isolated from higher plants - characterization of the double-membrane system," *Plant Cell Physiol.* 31, 975-985, 1990.

Gascoyne, Huang, Hughes, Wang, Pethig, Becker, "Manipulations of biological cells using travelling-wave dielectrophoresis," *Proc. 16th LEEE:Eng. Med. Biol. Soc.* 772-773, 1994a.

Gascoyne, Pethig, Burt, Becker, "Membrane changes accompanying the induced differentiation of friend murine erythroleukemia cells studied by dielectrophoresis," *Biochim. Biophys. Acta* 1149, 119-126, 1993.

Gascoyne, Wang, Becker, "Numerical analysis of the influence of experimental conditions on the accuracy of dielectric parameters derived from electrorotation measurements," *Bioelectrochem. Bioenerg.* 36, 115-125, 1994b.

Gefter *et al.*, *Somatic Cell Genet.,* 3: 231-236, 1977.

Gellert, Küstner, Hellwich, Kästner, in The VNR Concise Encyclopedia of Mathematics (Van Nostrand Reinhold, New York) pp 420-424, 1977.

Geohegan *et al.*, *Immunol. Comm* 7:1, 1978.

Gimsa, Marszalek, Loewe, Tsong, "Dielectrophoresis and electrorotation of neurospora slime and murine myeloma cells," *Biophys. J.* 60, 749-760, 1991.

Coding, 1986, *In* Monoclonal Antibodies: Principles and Practice, 2d ed., Orlando, Fla., Academic Press, 1986, pp. 60-61, 65-66, and 71-74.

Guatelli *et al., Proc. Natl. Acad. Sci. USA* 87:1874-1878, 1990.

Hagedorn, Fuhr, Müller, Gimsa, "Travelling-wave dielectrophoresis of microparticles," *Electrophoresis* 13:49-54, 1992.

Hölzel, Lamprecht, "Dielectric properties of yeast cells as determined by electrorotation," *Biochim. Biophys. Acta* 1104,195-200,1992.

Hölzel, Lamprecht, Mischel, "Characterisation of cells by dielectrophoretic techniques," in Physical Characterization of Biological Cells, eds. Schütt, W., Klinkmann, H., Lamprecht, I. & Wilson, T. (Verlag Gesundheit Gmh, Berlin), 1991.

Hu, Arnold, Zimmermann, "Alterations in the electrical properties of T and B lymphocyte membranes induced by mitogenic stimulation. Activation monitored by electro-rotation of single cells," *Biochim. Biophys. Acta* 1021, 191-200, 1990.

Huang, Hölzel, Pethig, Wang, "Differences in the AC electrodynamics of viable and non-viable yeast cells determined through combined dielectrophoresis and electrorotation studies," *Phys. Med. Biol.* 37, 1499-1517, 1992.

Huang, Wang, Tame, Pethig "Electrokinetic behaviour of colloidal particles in travelling electric fields: studies using yeast cells," *J. Phys. D: Appl. Phys.* 26:1528-1535, 1993

Irimajiri, Hanai, Inouye "A dielectric theory for "multi-stratified shell" model with its application to a lymphoma cell," *J. Theor. Biol.* 78 251-269, 1979.

Ithakissios *et al., Clin. Chem.,* 23, 2072-2079, 1977.

Jones, Electromechanics of Particles, Cambridge University Press, Cambridge, 1995.

Jones, Kallio, "Dielectrophoretic levitation of spheres and shells," *J. Electrostat.* 6 207-224, 1979.

Kaler and Jones, "Dielectrophoretic spectra of single cells determined by feedback-controlled levitation," *Biophys. J.* 57:173-182, 1990.

Kohler and Milstein, *Eur. J. Immunol.,* 6:511-519, 1976.

Kohler and Milstein, *Nature*, 256:495-497, 1975.

Landergren *et al., Science* 241:1077, 1988.

Lim, in Biomedical Applications of Microencapsulation, CRC Press, Boca Raton, 1984.

Lomeli *et al., Clin. Chem.* 35:1826, 1989.

*Margessi et al., Clin. Chim. Acta.,* 89, 455-460, 1978.

Masud, Washizu, Kawabata, "Movement of blood-cells in liquids by nonuniform traveling fields," *IEEE Trans. Ind. Appl.* 24:217-223, 1988.

Meuse, C.W., Fu, J., Conner, J.T., Plant, A.L. (1998). Construction of hybrid bilayers in air. Biophys. J. 74. 1388.)

Meuse, C.W.. Niaura, G., Lewis, M.L., Plant, A.L. (1998) Assessing the molecular structure of supported hybrid bilayer membranes with vibrational spectroscopies. Langmuir 14. 1604.

Milday *et al.*, *FEBS,* 170, 232-238, 1984.

Molday *et al., J. Immunol. Meth.,* 52, 353-367, 1982.

Molday *et al., Nature,* 268, 437, 1977.

Müller, Gerardino, Schnelle, Shirley, Bordoni, De Gasperis, Leoni, Fuhr, *J Phys D: Appl Phys* 29:340, 1996.

Nye *et al., Clin. Chim. Acta.*, 69, 387-396, 1976.

Oldenburg *et al.*, "Nanoengineering of optical resonances," Chemical Physics Letters 288 (1988) pp. 243-247 (May 1998).

Patterson *et al., Science* 260:976, 1993.

Pethig, Dielectric and Electronic Properties of Biological Materials, J. Wiley & Sons, New York, 1979.

Pethig, Markx , *Trends in Biotech* 15:426, 1997.

Plant, A.L. Self assembled phospholipid/alkanethiol biomimetic bilayers on gold. (1993) Langmuir 9: 2764-2767. [Online abstract]

Plant, A.L., Guegetchkeri, M., Yap, W. (1994) Supported phospholipid/alkanethiol biomimetic membranes: Insulating properties. Biophys. J. 67: 1126-1133. [Online abstract]

Plant, A.L, Gueguetchkeri, M. (1994) Planar phospholipid bilayer membranes formed form self-assembled alkanethiol monomers. Proceedings of the 13th Southern Biomedical Engineering Conference.

Plant, A.L., Brigham-Burke, M., O'Shannessy (1995) Phospholipid/alkanethiol bilayers for cell surface receptor studies by surface plasmon resonance. Anal. Biochem. 226:342-348. [Online abstract]

Pohl in Dielectrophoresis, Cambridge Universiy Press, Cambridge, 1978.

Rao, N.M., Plant, A.L., Hui, S.W., Silin, V., Wight, S. (1997) Characterization of biomimetic surfaces formed from cell membranes. Biophys. J. 73: 3066-3077. [Online abstract]

Rosenweig, *Scien. Amer.,* 252, 10,136-194 (1983).

Saiki *et al.*, *Science* 230:1350, 1985.

Saiki *et al., Science* 239:487, 1988.

Sauer, "Interaction-forces between microscopic particles in an external electromagnetic field," in Interactions Between Electromagnetic Fields and Cells ed A Chiabrera *et al* (New York: Plenum) pp 181-202, 1985.

Sukhorukov, Arnold, Zimmermann, "Hypotonically induced changes in the plasma membrane of cultured mammalian cells," *J. Membr. Biol.* 132,27-40,1993.

United States Patent No. 5,993,630.

United States Patent Application No. 09/249,955.

Walker *et al., Nucl. Acids Res.* 20:1691, 1992.

Wang, Huang, Becker, Gascoyne, "A Unified theory of dielectrophoresis and travelling wave dielectrophoresis," *J. Phys. D: Appl. Phys.* 27, 1571-1574, 1994a.

Wang, Huang, Burt, Markx, Pethig, "Selective dielectrophoretic confinement of bioparticles in potential energy wells," *J. Phys. D: Appl. Phys.* 26, 1278-1285, 1993.

Wang, Huang, Gascoyne, Becker, Hölzel, Pethig, "Changes in Friend murine erythroleukaemia cell membranes during induced differentiation determined by electrorotation," *Biochim. Biophys. Acta* 1193, 330-344, 1994c.

Wang, Hughes, Huang, Becker, Gascoyne, "Non-uniform spatial distributions of both the magnitude and phase of AC electric fields determine dielectrophoretic forces," *Biochim. Biophys. Acta*. 1243:185-194, 1994b.

Wang, Vykoukal, Becker, Gascoyne "Separation of polystyrene microbeads using DEP/G-FFF," *Biophysics Journal* 74:2689, 1998.

Widder *et al.*, Clin. Immunol. and Immunopath., 14,395-400, 1979.

Wu *et al., Proc. Natl. Acad. Sci.* USA 89:11769, 1992.

Wu, Sjodin, Sowers, "Distinct mechanical relaxation components in pairs of erythrocyte ghosts undergoing fusion," *Biophys. J*. 66, 114-119, 1994.

**Claims**

1. A dielectrically-engineered microparticle comprising a conductive core and an insulating self-assembled monolayer coating the conductive core, the monolayer having a thickness sufficient to render the microparticle maneuverable by dielectrophoresis.

**2.** The dielectrically-engineered microparticle of claim 1, wherein the conductive core comprises an insulator coated with a conducting shell.

**3.** The dielectrically-engineered microparticle of claim 1, wherein the conductive core comprises gold, silver, platinum, or copper.

**4.** A dielectrically-engineered microparticle label for binding to an analyte, the microparticle label comprising a dielectrically-engineered microparticle comprising a conductive core; an insulating layer coating the conductive core, the insulating layer having a thickness sufficient to render the microparticle label maneuverable by dielectrophoresis and a linking element coupled to the dielectrically-engineered microparticle.

**5.** A dielectrically-engineered microparticle label maneuverable by dielectrophoresis, comprising an insulating core coated with a conducting shell; a first self-assembled monolayer coating the conducting shell; and a second self-assembled monolayer coating the first self-assembled monolayer.

**6.** A method for detecting a complex within a sample, the method comprising admixing with the sample a dielectrically-engineered microparticle having a first dielectric property and comprising a conductive core, an insulating self-assembled layer having a thickness sufficient to render the microparticle maneuverable by dielectrophoresis, and a linking element; associating the dielectrically-engineered microparticle with a target analyte to form the complex, the complex having a second dielectric property; and detecting the complex by distinguishing between the first and second dielectric properties.

**7.** The method of claim 6, wherein the sample comprises blood, urine, saliva, amniotic fluid, biopsy, cell suspension, cell lysate, chromatographic fraction, or conditioned media.

**8.** The method of claim 6, wherein the sample comprises water, food, food processing, food distribution, mineral, or ore.

**9.** A method for manipulating a complex in a sample, the method comprising admixing with the sample a dielectrically-engineered microparticle comprising a conductive core, an insulating self-assembled layer coating the conductive core and having a thickness sufficient to render the microparticle maneuverable by dielectrophoresis, and a linking element; associating the dielectrically-engineered microparticle with the target analyte to form the complex; and manipulating the complex using dielectrophoresis.

**10.** The method of claim 9, wherein the sample comprises blood, urine, saliva, amniotic fluid, biopsy, cell suspension, cell lysate, chromatographic fraction, or conditioned media.

**11.** The method of claim 9, wherein the sample comprises water, food, food processing, food distribution, mineral, or ore.

**12.** The method of claim 9, wherein the manipulating comprises sorting.

**13.** The method of claim 9, wherein the manipulating comprises separating.

**14.** The method of claim 9, wherein the manipulating comprises purification of the sample.

**15.** The method of claim 9, wherein the manipulating comprises trapping.

**16.** A method for identifying one or more complexes within a sample, the method comprising admixing with the sample a plurality of dielectrically-engineered microparticles, each microparticle having a different dielectric property associating the plurality of dielectrically-engineered microparticles with one or more target analytes to form one or more complexes; and identifying the one or more complexes by distinguishing between the different dielectric properties.

**17.** The method of claim 16, wherein each the plurality of dielectrically-engineered microparticles comprise a conductive core and an insulating layer.

**18.** The dielectrically-engineered microparticle of claim 1 or the dielectrically-engineered microparticle label of claim 5, wherein the self-assembled monolayer comprises an alkanethiol self-assembled monolayer.

19. The dielectrically-engineered microparticle of claim 1 or the dielectrically-engineered microparticle label of claim 5, wherein the self-assembled monolayer or the second self-assembled monolayer comprises a phospholipid self-assembled monolayer, respectively.

20. The dielectrically-engineered microparticle label of claim 19, wherein the insulating core comprises polystyrene.

21. The dielectrically-engineered microparticle of claim 1 or the dielectrically-engineered microparticle label of claim 5, further comprising a linking element coupled to the microparticle and the label, respectively.

22. The dielectrically-engineered microparticle label of claim 21, further comprising a label coupled to the linking element.

23. The microparticle label of claim 22, wherein the label comprises a fluorescent marker, a chromophore, a luminescent marker, or an enzyme.

24. The method of claim 6, 9 or 17, wherein the insulating layer comprises one or more self-assembled monolayer layers.

25. The dielectrically-engineered microparticle of claim 21, the dielectrically-engineered microparticle label of claim 4 or 5, and the method of claim 6 or 9, wherein the linking element comprises an antibody, single chain antibody, peptide, hormone, nucleic acid sequence, therapeutic drug, antibiotic, or a chemically-reactive compound.

**Patentansprüche**

1. Dielektrisch hergestellter Mikropartikel, umfassend einen leitfähigen Kern und eine isolierende, selbst-assemblierte Einzelschicht, die den leitfähigen Kern ummantelt, wobei die Einzelschicht eine Dicke besitzt, die ausreicht, um den Mikropartikel durch Dielektrophorese manövrierfähig zu machen.

2. Dielektrisch hergestellter Mikropartikel gemäß Anspruch 1, wobei der leitfähige Kern einen Isolator umfasst, der mit einer leitenden Hülle ummantelt ist.

3. Dielektrisch hergestellter Mikropartikel gemäß Anspruch 1, wobei der leitfähige Kern Gold, Silber, Platin oder Kupfer umfasst.

4. Dielektrisch hergestellte Mikropartikel-Markierung zur Bindung an einen Analyten, wobei die Mikropartikel-Markierung einen dielektrisch hergestellten Mikropartikel umfasst, der einen leitfähigen Kern, eine isolierende-Schicht, die den leitfähigen Kern ummantelt, wobei die isolierende Schicht eine Dicke besitzt, die ausreichend ist, um die Mikropartikel-Markierung durch Dielektrophorese manövrierbar zu machen, und ein verbindendes Element umfasst, das an den dielektrisch hergestellten Mikropartikel gekoppelt ist.

5. Dielektrisch hergestellte, durch Dielektrophorese manövrierfähige Mikropartikel-Markierung, umfassend einen isolierenden Kern, der mit einer leitenden Hülle ummantelt ist, eine erste selbst-assemblierte Einzelschicht, welche die leitende Hülle ummantelt und eine zweite selbst-assemblierte Einzelschicht, welche die erste selbst-assemblierte Einzelschicht ummantelt.

6. Verfahren zum Nachweis eines Komplexes innerhalb einer Probe, wobei das Verfahren das Mischen der Probe mit einem dielektrisch hergestellten Mikropartikel umfasst, der eine erste dielektrische Eigenschaft besitzt und einen leitfähigen Kern, eine isolierende, selbst-assemblierte Schicht, die eine Dicke besitzt, die ausreicht, um den Mikropartikel durch Dielektrophorese manövrierfähig zu machen, und ein verbindendes Element umfasst; wobei das Verfahren weiterhin die Assoziierung des dielektrisch hergestellten Mikropartikels mit einem Ziel-Analyten umfasst, so dass der Komplex ausgebildet wird, wobei der Komplex eine zweite dielektrische Eigenschaft besitzt; und wobei das Verfahren weiterhin den Nachweis des Komplexes mittels der Unterscheidung zwischen der ersten und zweiten dielektrischen Eigenschaft umfasst.

7. Verfahren gemäß Anspruch 6, wobei die Probe Blut, Urin, Speichel, amniotische Flüssigkeit, eine Biopsie, eine Zellsuspension, ein Zelllysat, eine Chromatographiefraktion oder konditionierte Medien umfasst.

8. Verfahren gemäß Anspruch 6, wobei die Probe Wasser, Nahrungsmittel, Nahrungsmittelverarbeitungsprodukte, Nahrungsverteilungsprodukte, Mineralien oder Erz umfasst.

9. Verfahren zur Manipulation eines Komplexes in einer Probe, wobei das Verfahren das Mischen der Probe mit einem dielektrisch hergestellten Mikropartikel umfasst, der einen leitfähigen Kern, eine isolierende, selbst-assemblierte Schicht, die den leitfähigen Kern ummantelt und eine Dicke besitzt, die ausreicht, um den Mikropartikel durch Dielektrophorese manövrierfähig zu machen, und ein verbindendes Element umfasst; wobei das Verfahren weiterhin wobei das Verfahren weiterhin die Assoziierung des dielektrisch hergestellten Mikropartikels mit dem Ziel-Analyten umfasst, so dass der Komplex ausgebildet wird; und wobei das Verfahren weiterhin die Manipulation des Komplexes mittels Dielektrophorese umfasst.

10. Verfahren gemäß Anspruch 9, wobei die Probe Blut, Urin, Speichel, amniotische Flüssigkeit, eine Biopsie, eine Zellsuspension, ein Zelllysat, eine Chromatographiefraktion oder konditionierte Medien umfasst.

11. Verfahren gemäß Anspruch 9, wobei die Probe Wasser, Nahrungsmittel, Nahrungsmittelverarbeitungsprodukte, Nahrungsverteilungsprodukte, Mineralien oder Erz umfasst.

12. Verfahren gemäß Anspruch 9, wobei die Manipulation Sortierung umfasst.

13. Verfahren gemäß Anspruch 9, wobei die Manipulation Auftrennung umfasst.

14. Verfahren gemäß Anspruch 9, wobei die Manipulation Aufreinigung der Probe umfasst.

15. Verfahren gemäß Anspruch 9, wobei die Manipulation Einfangen umfasst.

16. Verfahren zur Identifizierung wenigstens eines Komplexes innerhalb einer Probe, wobei das Verfahren das Mischen der Probe mit einer Vielzahl von dielektrisch hergestellten Mikropartikeln umfasst, wobei jeder Mikropartikel eine unterschiedliche dielektrische Eigenschaft besitzt, welche die Vielzahl von dielektrisch hergestellten Mikropartikeln mit wenigstens einem Ziel-Analyten assoziiert, so dass wenigstens ein Komplex ausgebildet wird; und wobei das Verfahren weiterhin die Identifizierung des wenigstens einen Komplexes mittels Unterscheidung der unterschiedlichen dielektrischen Eigenschaften umfasst.

17. Verfahren gemäß Anspruch 16, wobei jeder aus der Vielzahl von dielektrisch hergestellten Mikropartikeln einen leitfähigen Kern und eine isolierende Schicht umfasst.

18. Dielektrisch hergestellter Mikropartikel gemäß Anspruch 1 oder dielektrisch hergestellte Mikropartikel-Markierung gemäß Anspruch 5, wobei die selbst-assemblierte Einzelschicht eine selbst-assemblierte Alkanthiol-Einzelschicht umfasst.

19. Dielektrisch hergestellter Mikropartikel gemäß Anspruch 1 oder dielektrisch hergestellte Mikropartikel-Markierung gemäß Anspruch 5, wobei die selbst-assemblierte Einzelschicht bzw. die zweite selbst-assemblierte Einzelschicht eine selbst-assemblierte Phospholipid-Einzelschicht umfasst.

20. Dielektrisch hergestellte Mikropartikel-Markierung gemäß Anspruch 19, wobei der isolierende Kern Polystyrol umfasst.

21. Dielektrisch hergestellter Mikropartikel gemäß Anspruch 1 oder dielektrisch hergestellte Mikropartikel-Markierung gemäß Anspruch 5, weiterhin umfassend ein verbindendes Element, das an den Mikropartikel bzw. die Markierung gekoppelt ist.

22. Dielektrisch hergestellte Mikropartikel-Markierung gemäß Anspruch 21, weiterhin umfassend eine Markierung, die an das verbindende Element gekoppelt ist.

23. Mikropartikel-Markierung gemäß Anspruch 22, wobei die Markierung eine fluoreszierende Markierung, ein Chromophor, eine lumineszierende Markierung oder ein Enzym umfasst.

**24.** Verfahren gemäß Anspruch 6, 9 oder 17, wobei die isolierende Schicht wenigstens eine selbst-assemblierte einzelschichtige Schicht umfasst.

**25.** Dielektrisch hergestellter Mikropartikel gemäß Anspruch 21, dielektrisch hergestellte Mikropartikel-Markierung gemäß Anspruch 4 oder 5 und Verfahren gemäß Anspruch 6 oder 9, wobei das verbindende Element einen Antikörper, einen Einzelkettenantikörper, ein Peptid, ein Hormon, eine Nukleinsäuresequenz, einen therapeutischen Wirkstoff, ein Antibiotikum oder eine chemisch reaktive Verbindung umfasst.

**Revendications**

**1.** Microparticule diélectriquement modifiée comprenant un noyau conducteur et une monocouche auto-assemblée isolante recouvrant le noyau conducteur, la monocouche ayant une épaisseur suffisante pour rendre la microparticule manoeuvrable par diélectrophorèse.

**2.** Microparticule diélectriquement modifiée selon la revendication 1, dans laquelle le noyau conducteur comprend un isolant recouvert d'une enveloppe conductrice.

**3.** Microparticule diélectriquement modifiée selon la revendication 1, dans laquelle le noyau conducteur comprend de l'or, de l'argent, du platine ou du cuivre.

**4.** Marqueur de microparticule diélectriquement modifiée pour lier une substance à analyser, le marqueur de microparticule comprenant une microparticule diélectriquement modifiée comprenant un noyau conducteur ; une couche isolante recouvrant le noyau conducteur, la couche isolante ayant une épaisseur suffisante pour rendre le marqueur de microparticule manipulable par diélectrophorèse et un élément de liaison couplé à la microparticule diélectriquement modifiée.

**5.** Marqueur de microparticule diélectriquement modifiée manoeuvrable par diélectrophorèse, comprenant un noyau isolant recouvert d'une enveloppe conductrice ; une première monocouche auto-assemblée recouvrant l'enveloppe conductrice ; et une seconde monocouche auto-assemblée recouvrant la première monocouche auto-assemblée.

**6.** Méthode pour détecter un complexe dans un échantillon, la méthode comprenant le mélange avec l'échantillon d'une microparticule diélectriquement modifiée ayant une première propriété diélectrique et comprenant un noyau conducteur, une couche auto-assemblée isolante ayant une épaisseur suffisante pour rendre la microparticule manipulable par diélectrophorèse, et un élément de liaison ; l'association de la microparticule diélectriquement modifiée à une substance à analyser cible pour former le complexe, le complexe ayant une seconde propriété diélectrique ; et la détection du complexe par distinction des première et seconde propriétés diélectriques.

**7.** Méthode selon la revendication 6, dans laquelle l'échantillon comprend du sang, de l'urine, de la salive, du liquide amniotique, une biopsie, suspension cellulaire, lysat cellulaire, fraction de chromatographie, ou milieu conditionné.

**8.** Méthode selon la revendication 6, dans laquelle l'échantillon comprend de l'eau, de l'aliment, de l'aliment transformé, de l'aliment distribué, du minéral, ou du minerai.

**9.** Méthode pour manipuler un complexe dans un échantillon, la méthode comprenant le mélange avec l'échantillon d'une microparticule diélectriquement modifiée comprenant un noyau conducteur, une couche auto-assemblée isolante recouvrant le noyau conducteur et ayant une épaisseur suffisante pour rendre la microparticule manipulable par diélectrophorèse, et un élément de liaison ; l'association de la microparticule diélectriquement modifiée avec la substance cible à analyser pour former le complexe ; et la manipulation du complexe par diélectrophorèse.

**10.** Méthode selon la revendication 9, dans laquelle l'échantillon comprend du sang, de l'urine, de la salive, du liquide amniotique, une biopsie, suspension cellulaire, lysat cellulaire, fraction de chromatographie, ou milieu conditionné.

**11.** Méthode selon la revendication 9, dans laquelle l'échantillon comprend de l'eau, de l'aliment, de l'aliment transformé, de l'aliment distribué, du minéral, ou du minerai.

**12.** Méthode selon la revendication 9, dans laquelle la manipulation comprend le tri.

**13.** Méthode selon la revendication 9, dans laquelle la manipulation comprend la séparation.

**14.** Méthode selon la revendication 9, dans laquelle la manipulation comprend la purification de l'échantillon.

**15.** Méthode selon la revendication 9, dans laquelle la manipulation comprend le piégeage.

**16.** Méthode d'identification d'un ou plusieurs complexes dans un échantillon, la méthode comprenant le mélange avec l'échantillon d'une pluralité de microparticules diélectriquement modifiées, chaque microparticule ayant une propriété diélectrique différente, l'association de la pluralité des microparticules diélectriquement modifiées à une ou plusieurs substances cibles à analyser pour former un ou plusieurs complexes ; et l'identification d'un ou plusieurs complexes par distinction des différentes propriétés diélectriques.

**17.** Méthode selon la revendication 16, dans laquelle chacune des microparticules de la pluralité de microparticules diélectriquement modifiées comprend un noyau conducteur et une couche isolante.

**18.** Microparticule diélectriquement modifiée selon la revendication 1 ou marqueur de microparticule diélectriquement modifiée selon la revendication 5, dans laquelle la monocouche auto-assemblée comprend une monocouche auto-assemblée à base d'alcanethiol.

**19.** Microparticule diélectriquement modifiée selon la revendication 1 ou marqueur de microparticule diélectriquement modifiée selon la revendication 5, dans laquelle la monocouche auto-assemblée ou la seconde monocouche auto-assemblée comprend respectivement une monocouche auto-assemblée à base de phospholipides.

**20.** Marqueur de microparticule diélectriquement modifiée selon la revendication 19, dans laquelle le noyau isolant comprend du polystyrène.

**21.** Microparticule diélectriquement modifiée selon la revendication 1 ou marqueur de microparticule diélectriquement modifiée selon la revendication 5, comprenant en outre un élément de liaison couplé à la microparticule et au marqueur, respectivement.

**22.** Marqueur de microparticule diélectriquement modifiée selon la revendication 21, comprenant en outre un marqueur couplé à l'élément de liaison.

**23.** Marqueur de microparticule selon la revendication 22, dans laquelle le marqueur comprend un marqueur fluorescent, un chromophore, un marqueur luminescent, ou une enzyme.

**24.** Méthode selon la revendication 6, 9 ou 17, dans laquelle la couche isolante comprend une ou plusieurs couches monocouches auto-assemblées.

**25.** Microparticule diélectriquement modifiée selon la revendication 21, marqueur de microparticule diélectriquement manipulée selon la revendication 4 ou 5, et méthode selon la revendication 6 ou 9, dans laquelle l'élément de liaison comprend un anticorps, un anticorps à chaîne unique, un peptide, une hormone, une séquence d'acide nucléique, une substance thérapeutique, un antibiotique, ou un composé réactif sur le plan chimique.

FIG. 1

FIG. 2B

FIG. 2A

FIG. 3

FIG. 4

FIG. 5

FIG. 6

FIG. 7

Legend:
$Re(f_{cm}) = -0.01$
$Re(f_{cm}) = -0.02$
$Re(f_{cm}) = -0.04$
$Re(f_{cm}) = -0.08$
$Re(f_{cm}) = -0.16$
$Re(f_{cm}) = -0.32$

x-axis: $\Delta(f_{cm})$ (0, -0.005, -0.01, -0.015, -0.02, -0.025)

y-axis: 10, 1.0, 0.1, 0.01, 0.001, 0.0001

**FIG. 8**

**FIG. 9A**

**FIG. 9B**

$$\sigma_{medium}=0.01\ S/m$$
$$\varepsilon_{medium}=78\times\varepsilon_0$$

$$\sigma_{core}=0.01\text{-}100\ S/m$$
$$\varepsilon_{core}=100\times\varepsilon_0$$

$$\varnothing_{core}=10\ \mu m$$

$$d_{shell}=10\ nm$$

$$\sigma_{shell}=1\times10^{-9}\ S/m$$
$$\varepsilon_{shell}=2\times\varepsilon_0$$

**FIG. 10A**

**FIG. 10B**

**FIG. 11A**

**FIG. 11B**

FIG. 12A

FIG. 12B

**FIG. 13A**

**FIG. 13B**

$\sigma_{medium}=0.01\text{-}10\ S/m$
$\varepsilon_{medium}=78\times\varepsilon_0$

$\sigma_{core}=6\times10^7\ S/m$
$\varepsilon_{core}=1000\times\varepsilon_0$

$\varnothing_{core}=10\mu m$

$d_{shell}=10\ nm$

$\sigma_{shell}=1\times10^{-9}\ S/m$
$\varepsilon_{shell}=2\times\varepsilon_0$

**FIG. 14A**

**FIG. 14B**

$\sigma_{medium}=0.01\ S/m$
$\varepsilon_{medium}=2\text{-}200\times\varepsilon_0$

$\sigma_{core}=6\times10^7\ S/m$
$\varepsilon_{core}=1000\times\varepsilon_0$

$\varnothing_{core}=10\ \mu m$

$d_{shell}=10\ nm$

$\sigma_{shell}=1\times10^{-9}\ S/m$
$\varepsilon_{shell}=2\times\varepsilon_0$

**FIG. 15A**

**FIG. 15B**

**FIG. 16**

POLYSTYRENE CORE

ALKANETHIOL SAM

GOLD SHELL

**FIG. 17**

EP 1 305 628 B1

FIG. 18

POLYSTYRENE CORE

CROSS-LINKED PHOSPHOLIPID SAM

ALKANETHIOL SAM

GOLD SHELL

FIG. 19

FIG. 20

EP 1 305 628 B1

FIG. 21

TARGET ANALYTE

PROTEIN PROBE (RECOGNITION ELEMENT)

POLYSTYRENE CORE

PHOSPHOLIPID SAM

ALKANETHIOL SAM

GOLD SHELL

FIG. 22